# EUROPÄISCHE PATENTSCHRIFT

(11) **EP 3 272 271 B1**
(45) Veröffentlichungstag und Bekanntmachung des Hinweises auf die Patenterteilung: **13.05.2020**
(21) Anmeldenummer: 17182098.8
(22) Anmeldetag: 19.07.2017
(51) Int. Cl.: A61B 1/005, A61B 1/267

(54) **ADAPTIVES LARYNGOSKOP UND ADAPTIVER SPATEL FÜR EIN LARYNGOSKOP**
ADAPTIVE LARYNGOSCOPE AND ADAPTIVE SPATULA FOR A LARYNGOSCOPE
LARYNGOSCOPE ADAPTATIF ET SPATULE ADAPTATIVE POUR UN LARYNGOSCOPE

(30) Priorität: 21.07.2016 DE 102016113498
(43) Veröffentlichungstag der Anmeldung: 24.01.2018
(73) Patentinhaber: Karl Storz SE & Co. KG, 78532 Tuttlingen (DE)
(72) Erfinder: Aleckner, Martina, 78532 Tuttlingen (DE)

(56) Entgegenhaltungen:
- DE-T2- 60 221 889
- DE-U1- 29 921 801
- FR-A1- 2 821 736
- US-A- 5 584 795
- US-A- 6 053 166
- US-A1- 2010 261 968
- US-A1- 2011 196 204
- US-A1- 2013 310 650
- US-A1- 2015 031 957

## Beschreibung

Die vorliegende Erfindung ist bezogen auf ein adaptives Laryngoskop, insbesondere ein adaptives Intubationslaryngoskop oder ein adaptives Laryngoskop für die Laryngoskopie, die Larynxchirurgie oder andere Aufgaben innerhalb der Hals-Nasen-Ohren-Heilkunde bzw. Oto-Rhino-Laryngologie (engl.: Otorhinolaryngology). Die vorliegende Erfindung ist ferner auf einen adaptiven Spatel (engl.: blade) für ein solches Laryngoskop oder für eine Intubationsvorrichtung bezogen.

Zur endotrachealen Intubation in Anästhesie, Notfallmedizin und Intensivmedizin sowie der Chirurgie des Kehlkopfs (Larynx) werden für eine Intubation oder für chirurgische Maßnahmen ein unverlegter Zugang zum Kehlkopf, den Stimmbändern und letztlich oft auch der Trachea benötigt. Dabei dient ein Laryngoskop dazu, die Zunge nach vorne bzw. rostral zu schieben. Ein Laryngoskop umfasst in der Regel einen mehr oder weniger gekrümmten Spatel, an dessen proximalem Ende in näherungsweise rechtem Winkel ein Griff angeordnet ist.

Um eine Anpassung an die Anatomie des Patienten zu ermöglichen, ist der Spatel in der Regel austauschbar. In einem Intubationsset befindet sich eine Vielzahl von Spateln unterschiedlicher Länge und unterschiedlicher Krümmung. Für verschiedene Anwendungen und/oder unterschiedliche Vorlieben des medizinischen Personals stehen ferner unterschiedliche Bauformen zur Verfügung, beispielsweise nach Macintosh, Miller, Dörges und McCoy, letztere mit einem bewegbaren distalen Ende.

Ein Laryngoskop mit einem verformbaren distalen Ende ist auch in WO 97/30626 (später auch veröffentlicht als US 6,174,281 B1) beschrieben. Der Spatel 4 des Laryngoskops weist in einem mittleren Abschnitt 14 mehrere Schlitze 40 auf. Die Schlitze 40 unterteilen den mittleren Abschnitt 14 in Segmente 42, die nur durch schmale, als Festkörpergelenke wirkende Stege miteinander verbunden sind.

In EP 1 040 999 A2 ist ein Bauteil zur Aufnahme von Kräften beschrieben, bei dem Streben 11, 11a gegenüberliegende Bereiche einer Außenhaut 12, 12a miteinander verbinden. In EP 2 241 403 A1 ist ein Manipulatorwerkzeug mit zwei flexiblen Wangen 8, 10 beschrieben. Die Wangen 8, 10 sind am distalen Ende 6 des Manipulatorwerkzeugs 1 unmittelbar und im Übrigen durch mehrere Scharnierelemente 20 miteinander verbunden.

In DE 10 2007 026 721 A1 ist ein medizinisches Greifwerkzeug zum Halten von Körperbestandteilen beschrieben. Das medizinische Greifwerkzeug 1 umfasst mehrere Branchen 1 mit jeweils zwei gegenüberliegenden Wangen, zwischen denen verbindende Elemente verlaufen.

In DE 10 2005 010 380 B4 ist ein Greifwerkzeug mit selbstadaptiver Kinematik beschrieben.

In der DE2992180U ist ein Laryngoskop beschrieben mit mehreren, nur proximal miteinander verbundenen Fingern aus mehreren Gliedern.

Eine Aufgabe der vorliegenden Erfindung besteht darin, einen verbesserten adaptiven Spatel für ein Laryngoskop und ein verbessertes adaptives Laryngoskop zu schaffen.

Diese Aufgabe wird durch die Gegenstände der unabhängigen Ansprüche gelöst. Weiterbildungen sind in den abhängigen Ansprüchen angegeben.

Ein adaptiver Spatel für ein Laryngoskop umfasst ein proximales Ende, ein distales Ende, eine zwischen dem proximalen Ende und dem distalen Ende des adaptiven Spatels angeordnete erste Kette aus einer Mehrzahl von Kettengliedern, die jeweils paarweise gelenkig verbunden sind, eine zwischen dem proximalen Ende und dem distalen Ende des adaptiven Spatels angeordnete zweite Kette aus einer Mehrzahl von Kettengliedern, die jeweils paarweise gelenkig verbunden sind, und ein Abstandsbauteil, wobei ein erstes Ende des Abstandsbauteils gelenkig mit einem der Kettenglieder der ersten Kette verbunden ist, wobei ein zweites Ende des Abstandsbauteils gelenkig mit einem der Kettenglieder der zweiten Kette verbunden ist, wobei eine oder mehrere oder alle der gelenkigen Verbindungen zwischen den Kettengliedern der ersten Kette, den Kettengliedern der zweiten Kette und dem Abstandsbauteil ein formschlüssiges Gelenk umfassen.

Der adaptive Spatel ist insbesondere zur Bildung eines Laryngoskops vorgesehen und ausgebildet, das zur Intubation oder für die Mikrolarynxchirurgie oder für andere Aufgaben innerhalb der Oto-Rhino-Laryngologie verwendbar ist. Das proximale Ende des adaptiven Spatels kann mit einem Griffbauteil dauerhaft - insbesondere für die gesamte vorgesehene Lebensdauer des Laryngoskops - und nicht zerstörungsfrei trennbar mechanisch verbunden sein. Insbesondere kann ein proximales Ende des adaptiven Spatels teilweise oder ganz mit dem Griffbauteil einstückig ausgebildet sein, beispielsweise als gleichzeitig gefertigtes Gussteil aus Kunststoff, Metall oder einem anderen hinreichend elastischen Material. Alternativ kann am proximalen Ende des adaptiven Spatels eine Kupplungseinrichtung (beispielsweise in der Art einer Bajonett- bzw. Renkverbindung, einer Schraubverbindung, einer Rastverbindung) zur einmaligen oder wiederholbaren und zerstörungsfrei lösbaren Kopplung mit einem Griffbauteil vorgesehen sein.

Das distale Ende des adaptiven Spatels ist insbesondere zum Einführen in den Rachen und zum Annähern an den Kehlkopf eines Patienten vorgesehen und ausgebildet.

Die erste Kette und die zweite Kette verbinden jeweils insbesondere unmittelbar oder mittelbar das proximale Ende und das distale Ende des adaptiven Spatels. Die erste Kette und die zweite Kette sind insbesondere näherungsweise parallel angeordnet, wobei ihr Abstand von proximal nach distal abnimmt.

Die erste Kette ist insbesondere vorgesehen und ausgebildet, um bei der vorgesehenen und bestimmungsgemäßen Verwendung des adaptiven Spatels an der Zunge eines Patienten anzuliegen. Dazu sind die Kettenglieder der ersten Kette insbesondere jeweils breit und mit einer ebenen oder glatten oder weitgehend glatten und zur Anlage an der Zunge vorgesehenen Oberfläche ausgebildet.

Die erste Kette umfasst zwei, drei, vier, fünf, sechs oder mehr Kettenglieder. Die Kettenglieder der ersten Kette können untereinander gleich oder ähnlich sein. Insbesondere nimmt die Breite der Kettenglieder der ersten Kette von proximal nach distal ab.

Jedes einzelne Kettenglied der ersten Kette ist insbesondere in sich steif, d. h. bei den bei der vorgesehenen Verwendung des adaptiven Spatels auftretenden Kräften und Momenten nicht oder nicht wesentlich verformbar. Alternativ können ein Kettenglied oder mehrere Kettenglieder der ersten Kette flexibel d. h. bei den bei der vorgesehenen Verwendung des adaptiven Spatels auftretenden Kräften und Momenten elastisch und/oder plastisch verformbar ausgebildet sein.

Die zweite Kette ist vorgesehen, ausgebildet und angeordnet, um bei der vorgesehenen Verwendung des adaptiven Spatels nicht an der Zunge des Patienten anzuliegen. Die Anzahl der Kettenglieder der zweiten Kette entspricht insbesondere der Anzahl der Kettenglieder der ersten Kette.

Jedes einzelne Kettenglied der zweiten Kette ist insbesondere in sich steif, d. h. bei den bei der vorgesehenen Verwendung des adaptiven Spatels auftretenden Kräften und Momenten nicht oder nicht wesentlich verformbar. Alternativ können ein Kettenglied oder mehrere Kettenglieder der zweiten Kette flexibel, d. h. bei den bei der vorgesehenen Verwendung des adaptiven Spatels auftretenden Kräften und Momenten elastisch und/oder plastisch verformbar sein.

Der adaptive Spatel kann ein oder mehrere Abstandsbauteile aufweisen. Insbesondere ist die Anzahl der Abstandsbauteile genauso groß wie die Anzahl der gelenkigen Verbindungen zwischen benachbarten Kettengliedern der zweiten Kette und/oder genauso groß wie die Anzahl der gelenkigen Verbindungen zwischen benachbarten Kettengliedern der ersten Kette. Wenn der adaptive Spatel mehrere Abstandsbauteile aufweist, weisen diese insbesondere unterschiedliche Längen auf, wobei die Länge der Abstandsbauteile von proximal nach distal abnimmt.

Das Abstandsbauteil oder die Abstandsbauteile können jeweils steif, d. h. bei den bei der vorgesehenen Anwendung auftretenden Kräften und Momenten nicht oder nicht wesentlich verformbar, ausgebildet sein. Alternativ können das Abstandsbauteil oder eines oder mehrere der Abstandsbauteile flexibel, d. h. bei den bei der vorgesehenen Anwendung des adaptiven Spatels auftretenden Kräften und Momenten elastisch und/oder plastisch verformbar, sein. Eine Flexibilität von einem oder mehreren Abstandsbauteilen kann die Kraft, die mit dem adaptiven Spatel übertragen oder ausgeübt werden kann, begrenzen.

Das erste Ende des Abstandsbauteils oder das erste Ende von einem von mehreren Abstandsbauteilen ist insbesondere mit einem Ende eines Kettenglieds der ersten Kette oder mit zwei benachbarten Enden benachbarter Kettenglieder der ersten Kette unmittelbar oder mittelbar gelenkig verbunden. Das zweite Ende des Abstandsbauteils oder das zweite Ende von einem von mehreren Abstandsbauteilen ist insbesondere mit einem Ende eines Kettenglieds der zweiten Kette oder mit zwei benachbarten Enden zweier benachbarter Kettenglieder der zweiten Kette unmittelbar oder mittelbar gelenkig verbunden.

Der adaptive Spatel kann zwei zweite Ketten, die im Wesentlichen parallel angeordnet sind, deren Abstand jedoch insbesondere von proximal nach distal abnimmt, umfassen. In diesem Fall sind insbesondere jeweils zwei Abstandsbauteile parallel oder im Wesentlichen parallel angeordnet, die jeweils ein oder zwei Kettenglieder von jeder der beiden zweiten Ketten mit gegenüberliegenden Rändern der ersten Kette verbinden.

Die beiden zweiten Ketten und die Abstandsbauteile sowie die erste Kette sind insbesondere spiegelsymmetrisch zu einer Symmetrieebene angeordnet. In einer Schnittebene orthogonal zu dieser Symmetrieebene weist der adaptive Spatel einen im Wesentlichen U-förmigen Querschnitt auf.

Alternativ kann der adaptive Spatel mit einer zweiten Kette oder mit mehreren zweiten Ketten asymmetrisch ausgebildet sein, so dass er zu keiner Ebene spiegelsymmetrisch ist.

Formschlüssige Gelenke sind Gelenke, die nicht auf Elastizität eines Bauteils oder eines Bereichs eines Bauteils (beispielsweise eines Foliengelenks) beruhen, sondern auf formschlüssiger Führung zweier Bauteile aneinander. Formschlüssige Gelenke umfassen insbesondere Wälz- oder Gleitlager mit je zwei korrespondierenden Lagerflächen, die unmittelbar aneinander gleiten oder zwischen denen Wälzkörper angeordnet sind.

Die Verwendung von einem oder mehreren formschlüssigen Gelenken zwischen Kettengliedern der ersten Kette und/oder zwischen Kettengliedern der zweiten Kette und/oder zwischen dem ersten Ende eines Abstandsbauteils und einem oder mehreren Kettengliedern der ersten Kette und/oder zwischen dem zweiten Ende des Abstandsbauteils und einem oder mehreren Kettengliedern der zweiten Kette kann eine spiel- und reibungsarme gelenkige Verbindung von Bauteilen des adaptiven Spatels ermöglichen. Der Spatel kann dadurch auf sehr genau definierte Weise adaptiv sein, sich also an die Oberfläche und die Verformbarkeit der Anatomie eines Patienten anpassen. Dabei sind - anders als bei einem herkömmlichen adaptiven Spatel, der ausschließlich Festkörpergelenke bzw. stoffschlüssige Gelenke aufweist, keine Kompromisse hinsichtlich des Materials erforderlich. Die Kettenglieder und das Abstandsbauteil oder die Abstandsbauteile können aus beliebigen Materialien, insbesondere auch aus sehr steifen Materialien mit hohem Elastizitätsmodul, aus spröden Materialien und aus Materialien mit einer niedrigen Elastizitätsgrenze gebildet sein.

Bei einem adaptiven Spatel, wie er hier beschrieben ist, ist insbesondere zumindest ein Teil der gelenkigen Verbindungen durch einachsige Schwenkgelenke gebildet.

Ein einachsiges Schwenkgelenkt ist ein Gelenk, das lediglich einen Freiheitsgrad aufweist, und bei dem eines der beiden durch das Schwenkgelenk miteinander verbundenen Bauteile relativ zu dem anderen Bauteil lediglich um eine vorbestimmte Schwenkachse schwenkbar ist. Diese Schwenkbarkeit um die vorbestimmte Schwenkachse ist insbesondere auf einen vorbestimmten Winkelbereichs begrenzt.

Insbesondere sind alle gelenkigen Verbindungen des adaptiven Spatels durch einachsige Schwenkgelenke gebildet. Die vorbestimmten Schwenkachsen aller Gelenke sind insbesondere parallel zueinander oder schneiden sich in einem Punkt. In dem oben beschriebenen Fall eines adaptiven Spatels mit zwei zweiten Ketten, die spiegelsymmetrisch zu einer Symmetrieebene angeordnet sind, sind die Schwenkachsen insbesondere orthogonal zu dieser Symmetrieebene.

Bei einem adaptiven Spatel, wie er hier beschrieben ist, umfassen insbesondere eine gelenkige Verbindung zwischen zwei benachbarten Kettenglieder der ersten Kette ein formschlüssiges Gelenk und eine gelenkige Verbindung zwischen zwei benachbarten Kettengliedern der zweiten Kette ein formschlüssiges Gelenk.

Bei einem adaptiven Spatel, wie er hier beschrieben ist, umfassen insbesondere mehrere gelenkige Verbindungen zwischen jeweils zwei benachbarten Kettengliedern der ersten Kette jeweils ein formschlüssiges Gelenk und mehrere gelenkige Verbindungen zwischen jeweils zwei benachbarten Kettengliedern der zweiten Kette jeweils ein formschlüssiges Gelenk.

Bei einem adaptiven Spatel, wie er hier beschrieben ist, umfassen insbesondere mehrere gelenkige Verbindungen zwischen Abstandsbauteilen und Kettengliedern der ersten Kette jeweils ein formschlüssiges Gelenk.

Bei einem adaptiven Spatel, wie er hier beschrieben ist, umfassen insbesondere mehrere gelenkige Verbindungen zwischen Abstandsbauteilen und Kettengliedern der zweiten Kette jeweils ein formschlüssiges Gelenk.

Bei einem adaptiven Spatel, wie er hier beschrieben ist, umfasst das formschlüssige Gelenk insbesondere eine in eine Bohrung in einem vorbestimmten Kettenglied eingesetzte Welle, wobei die Welle zumindest entweder durch eine Übermaßpassung kraftschlüssig in der Bohrung in dem vorbestimmten Kettenglied gehalten ist oder durch eine Schweißnaht mit dem vorbestimmten Kettenglied verbunden ist.

Bei einem adaptiven Spatel, wie er hier beschrieben ist, greift insbesondere ein erstes Kettenglied in eine Ausnehmung in einem zweiten Kettenglied, wobei das formschlüssige Gelenk eine in eine Bohrung in dem zweiten Kettenglied eingesetzten Welle umfasst, wobei die Welle durch die Ausnehmung in dem zweiten Kettenglied und innerhalb der Ausnehmung durch das erste Kettenglied greift, wobei die Welle zumindest entweder durch eine Übermaßpassung kraftschlüssig in der Bohrung in dem zweiten Kettenglied gehalten ist oder durch eine Schweißnaht mit dem zweiten Kettenglied verbunden ist.

Das vorbestimmte Kettenglied bzw. das erste Kettenglied und das zweite Kettenglied sind insbesondere Teil der ersten Kette des adaptiven Spatels. Alternativ oder zusätzlich kann auch ein Gelenk an der zweiten Kette des adaptiven Spatels so ausgestaltet sein.

Die Welle weist insbesondere die Gestalt eines Stifts auf, der an einem Ende leicht konisch ausgebildet sein kann, um bei der Montage des adaptiven Spatels ein Einsetzen in die Bohrung zu vereinfachen. Die Bohrung ist insbesondere eine Sackbohrung in dem vorbestimmten Kettenglied bzw. in dem zweiten Kettenglied.

Die Übermaßpassung umfasst einen Abschnitt oder Bereich der Bohrung, in dem der Innendurchmesser der Bohrung vor dem Einsetzen der Welle, d. h. im mechanisch spannungsfreien Zustand, kleiner ist als der Außendurchmesser der Welle in dem dazu korrespondierenden Bereich vor dem Einsetzen in die Bohrung, d. h. im mechanisch spannungsfreien Zustand.

Bei der Montage wird die Welle in die Bohrung gepresst, wobei in der Welle und in der Umgebung der Bohrung mechanische Spannungen entsteht. Die resultierenden Rückstellkräfte halten die Welle kraft- bzw. reibschlüssig in der Bohrung.

Bei Ausgestaltung des adaptiven Spatels derart, dass die Welle durch eine Ausnehmung in dem zweiten Kettenglied und innerhalb der Ausnehmung durch das benachbarte erste Kettenglied greift, ist die Übermaßpassung insbesondere nicht nahe einer äußeren Oberfläche des zweiten Kettenglieds angeordnet. Stattdessen liegt die Übermaßpassung insbesondere in einem Bereich der Bohrung, der von der äußeren Oberfläche des zweiten Kettenglieds beabstandet ist, vor.

Im Fall einer Ausgestaltung der Bohrung als Sackbohrung mit einem offenen Ende und einem geschlossenen Ende sind die Ausnehmung in dem zweiten Kettenglied insbesondere zwischen dem offenen Ende und dem geschlossenen Ende der Sackbohrung und die Übermaßpassung zwischen der Ausnehmung und dem geschlossenen Ende der Sackbohrung angeordnet. Die Übermaßpassung kann ein Eindringen von Schmutz in Form eines Fluids oder eines Festkörpers in den Bereich der Sackbohrung zwischen der Ausnehmung und ihrem geschlossenen Ende verhindern.

Auch im Fall einer Übermaßpassung kann zusätzlich eine Schweißnaht oder eine andere stoffschlüssige Fügung die Welle mit dem zweiten Kettenglied verbinden. Eine Schweißnaht ist insbesondere an dem offenen Ende der Bohrung an einer äußeren Oberfläche des zweiten Kettenglieds vorgesehen. Die Schweißnaht umschließt insbesondere das Ende der Welle kreisförmig.

Bei einem adaptiven Spatel, wie er hier beschrieben ist, sind Kettenglieder der ersten Kette insbesondere im Wesentlichen plattenförmig mit rechteckigem oder trapezförmigem Grundriss.

Insbesondere sind alle Kettenglieder der ersten Kette jeweils im Wesentlichen plattenförmig mit rechteckigem oder trapezförmigem Grundriss. Ein trapezförmiger Grundriss plattenförmiger Kettenglieder ermöglicht beispielsweise eine von proximal nach distal abnehmende Breite des adaptiven Spatels.

Bei einem adaptiven Spatel, wie er hier beschrieben ist, weisen insbesondere zumindest entweder ein Kettenglied der ersten Kette oder ein Kettenglied der zweiten Kette oder das Abstandsbauteil Metall auf.

Insbesondere weisen alle Kettenglieder der ersten Kette und/oder alle Kettenglieder der zweiten Kette und/oder alle Abstandsbauteile Metall auf. Insbesondere sind alle Kettenglieder der ersten Kette und/oder alle Kettenglieder der zweiten Kette und/oder alle Abstandsbauteile aus Metall gebildet.

Die Verwendung von Metall kann eine große Steifheit der einzelnen Kettenglieder und Abstandsbauteile und damit eine sehr definierte, nämlich ausschließlich oder fast ausschließlich durch die gelenkigen Verbindungen definierte Verformbarkeit des adaptiven Spatels ermöglichen. Dies kann eine definiertere oder besser dosierte und kontrolliertere Anwendung von Kraft durch medizinisches Personal auf den Patienten ermöglichen.

Ein adaptiver Spatel, wie er hier beschrieben ist, umfasst insbesondere ferner eine Mehrfachgelenkeinrichtung, in die eine gelenkige Verbindung zwischen zwei Kettengliedern der ersten Kette und eine gelenkige Verbindung zwischen einem Kettenglied der ersten Kette und dem Abstandsbauteil integriert ist.

Die Mehrfachgelenkeinrichtung ist insbesondere mit der beschriebenen Übermaßpassung zwischen einer Welle und einer Bohrung, in die die Welle eingesetzt ist, ausgestaltet.

In die Mehrfachgelenkeinrichtung sind insbesondere die gelenkige Verbindung zwischen zwei Kettengliedern der ersten Kette und die gelenkige Verbindung zwischen einem Kettenglied der ersten Kette und dem Abstandsbauteil innerhalb eines kleinen Bauraums integriert. Das Volumen des Bauraums der Mehrfachgelenkeinrichtung ist insbesondere nicht größer oder nicht wesentlich größer als die Summe der Volumina zweier einzelner einfacher Gelenkeinrichtungen. Die Mehrfachgelenkeinrichtung umfasst insbesondere zwei einzelne Gelenke, nämlich ein Gelenk zwischen zwei Kettengliedern der ersten Kette und ein Gelenk zwischen einem Kettenglied der ersten Kette und dem Abstandsbauteil oder ein Gelenk zwischen dem Abstandsbauteil und einem ersten Kettenglied der ersten Kette und ein Gelenk zwischen dem Abstandsbauteil und einem zweiten Kettenglied der ersten Kette.

Eine Mehrfachgelenkeinrichtung kann hinsichtlich der Fertigungskosten, des erforderlichen Bauraums, der adaptiven Funktion und der mechanischen Robustheit vorteilhaft sein.

Bei einem adaptiven Spatel, wie er hier beschrieben ist, umfasst die Mehrfachgelenkeinrichtung insbesondere eine Welle oder einen Achszapfen mit einer ersten Lagerfläche und einer zweiten Lagerfläche, wobei die erste Lagerfläche und die zweite Lagerfläche Bestandteile gelenkiger Verbindungen des Abstandsbauteils mit zwei Kettengliedern der ersten Kette sind, oder wobei die erste Lagerfläche Bestandteil der gelenkigen Verbindung zwischen zwei Kettengliedern der ersten Kette und die zweite Lagerfläche Bestandteil der gelenkigen Verbindung zwischen einem Kettenglied der ersten Kette und dem Abstandsbauteil ist.

Die Mehrfachgelenkeinrichtung umfasst insbesondere ferner eine zu der ersten Lagerfläche korrespondierende dritte Lagerfläche, wobei die erste Lagerfläche und die dritte Lagerfläche aneinander gleiten oder mehrere Wälzkörper zwischen der ersten Lagerfläche und der dritten Lagerfläche angeordnet sind. Die Mehrfachgelenkeinrichtung umfasst insbesondere ferner eine zu der zweiten Lagerfläche korrespondierende vierte Lagerfläche, wobei die zweite Lagerfläche und die vierte Lagerfläche aneinander gleiten oder mehrere Wälzkörper zwischen der zweiten Lagerfläche und der vierten Lagerfläche angeordnet sind.

Die erste Lagerfläche und die zweite Lagerfläche an der Welle oder dem Achszapfen der Mehrfachgelenkeinrichtung können fluchten oder ineinander übergehen oder durch verschiedene Teilbereiche eines kreiszylindrischen Oberflächenbereichs der Welle oder des Achszapfens gebildet sein. Alternativ können die erste Lagerfläche und die zweite Lagerfläche beispielsweise durch eine Stufe voneinander getrennt sein, wobei die erste Lagerfläche und die zweite Lagerfläche unterschiedliche Krümmungsradien aufweisen können.

Die Welle oder der Achszapfen kann starr mit einem ersten der Kettenglieder der ersten Kette verbunden sein. In diesem Fall sind eine zu der ersten Lagerfläche korrespondierende dritte Lagerfläche an einem zweiten Kettenglied der ersten Kette und eine zu der zweiten Lagerfläche korrespondierende vierte Lagerfläche an dem Abstandsbauteil vorgesehen. Alternativ kann die Welle oder der Achszapfen starr mit dem Abstandsbauteil verbunden sein. In diesem Fall sind die zu der ersten Lagerfläche korrespondierende dritte Lagerfläche an einem ersten Kettenglied der ersten Kette und die zu der zweiten Lagerfläche korrespondierende vierte Lagerfläche an einem zweiten Kettenglied der ersten Kette angeordnet.

Ein adaptiver Spatel, wie er hier beschrieben ist, umfasst insbesondere ferner eine Mehrfachgelenkeinrichtung, in die eine gelenkige Verbindung zwischen zwei Kettengliedern der zweiten Kette und eine gelenkige Verbindung zwischen einem Kettenglied der zweiten Kette und dem Abstandsbauteil integriert ist.

In die Mehrfachgelenkeinrichtung sind insbesondere die gelenkige Verbindung zwischen zwei Kettengliedern der zweiten Kette und die gelenkige Verbindung zwischen einem Kettenglied der zweiten Kette und dem Abstandsbauteil innerhalb eines kleinen Bauraums integriert. Das Volumen des Bauraums der Mehrfachgelenkeinrichtung ist insbesondere nicht größer oder nicht wesentlich größer als die Summe der Volumina zweier einzelner einfacher Gelenkeinrichtungen. Die Mehrfachgelenkeinrichtung umfasst insbesondere zwei einzelne Gelenke, nämlich ein Gelenk zwischen zwei Kettengliedern der zweiten Kette und ein Gelenk zwischen einem Kettenglied der zweiten Kette und dem Abstandsbauteil oder ein Gelenk zwischen dem Abstandsbauteil und einem ersten Kettenglied der zweiten Kette und ein Gelenk zwischen dem Abstandsbauteil und einem zweiten Kettenglied der zweiten Kette.

Bei einem adaptiven Spatel, wie er hier beschrieben ist, umfasst die Mehrfachgelenkeinrichtung insbesondere eine Welle oder einen Achszapfen oder einen Stift mit einer ersten Lagerfläche und einer zweiten Lagerfläche, wobei die erste Lagerfläche und die zweite Lagerfläche Bestandteile gelenkiger Verbindungen des Abstandsbauteils mit zwei Kettengliedern der zweiten Kette sind, oder wobei die erste Lagerfläche Bestandteil der gelenkigen Verbindung zwischen zwei Kettengliedern der zweiten Kette und die zweite Lagerfläche Bestandteil der gelenkigen Verbindung zwischen einem Kettenglied der zweiten Kette und dem Abstandsbauteil ist.

Die Mehrfachgelenkeinrichtung umfasst insbesondere ferner eine zu der ersten Lagerfläche korrespondierende dritte Lagerfläche, wobei die erste Lagerfläche und die dritte Lagerfläche aneinander gleiten oder mehrere Wälzkörper zwischen der ersten Lagerfläche und der dritten Lagerfläche angeordnet sind. Die Mehrfachgelenkeinrichtung umfasst insbesondere ferner eine zu der zweiten Lagerfläche korrespondierende vierte Lagerfläche, wobei die zweite Lagerfläche und die vierte Lagerfläche aneinander gleiten oder mehrere Wälzkörper zwischen der zweiten Lagerfläche und der vierten Lagerfläche angeordnet sind.

Die erste Lagerfläche und die zweite Lagerfläche an der Welle oder dem Achszapfen der Mehrfachgelenkeinrichtung können fluchten oder ineinander übergehen oder durch verschiedene Teilbereiche eines kreiszylindrischen Oberflächenbereichs der Welle oder des Achszapfens gebildet sind. Alternativ können die erste Lagerfläche und die zweite Lagerfläche beispielsweise durch eine Stufe voneinander getrennt sein, wobei die erste Lagerfläche und die zweite Lagerfläche unterschiedliche Krümmungsradien aufweisen können.

Die Welle oder der Achszapfen kann starr mit einem ersten der Kettenglieder der zweiten Kette verbunden sein. In diesem Fall sind eine zu der ersten Lagerfläche korrespondierende dritte Lagerfläche an einem zweiten Kettenglied der zweiten Kette und eine zu der zweiten Lagerfläche korrespondierende vierte Lagerfläche an dem Abstandsbauteil vorgesehen. Alternativ kann die Welle oder der Achszapfen starr mit dem Abstandsbauteil verbunden sein. In diesem Fall sind die zu der ersten Lagerfläche korrespondierende dritte Lagerfläche an einem ersten Kettenglied der zweiten Kette und die zu der zweiten Lagerfläche korrespondierende vierte Lagerfläche an einem zweiten Kettenglied der zweiten Kette angeordnet.

Bei einem adaptiven Spatel, wie er hier beschrieben ist, sind insbesondere Lagerflächen an zwei verschiedenen Kettengliedern und eine Lagerfläche an dem Abstandsbauteil koaxial angeordnet.

Die Lagerflächen an zwei verschiedenen Kettengliedern und die Lagerfläche an dem Abstandsbauteil sind insbesondere zumindest teilweise ineinander angeordnet, so dass eine Lagerfläche die beiden anderen Lagerflächen umschließt. Dabei kann eine erste der drei Lagerflächen eine zweite der drei Lagerflächen umschließen und von einer dritten der drei Lagerflächen umschlossen werden. Beispielsweise ist an einem der zwei Kettenglieder eine Hülse vorgesehen, an deren innerer Oberfläche und an deren äußerer Oberfläche je eine Lagerfläche vorgesehen sind, wobei eine Welle oder ein Achszapfen an einem weiteren Kettenglied in der Hülse geführt ist und die Hülse ihrerseits in einer Bohrung an dem Abstandsbauteil geführt ist.

Ein adaptiver Spatel, wie er hier beschrieben ist, umfasst insbesondere ferner korrespondierende Anschlagflächen an zwei miteinander gelenkig verbundenen Kettengliedern, wobei die korrespondierenden Anschlagflächen so angeordnet sind, dass ein mechanischer Kontakt der korrespondierenden Anschlagflächen eine Schwenkbewegung der Kettenglieder, an denen die korrespondierenden Anschlagflächen angeordnet sind, relativ zueinander begrenzt.

Die korrespondierenden Anschlagflächen sind insbesondere in eine gelenkige Verbindung zwischen den beiden Kettengliedern integriert oder in unmittelbarer Nähe zu der gelenkigen Verbindung zwischen den Kettengliedern angeordnet. Die korrespondierenden Anschlagflächen sind insbesondere an zwei miteinander gelenkig verbundenen Kettengliedern der ersten Kette vorgesehen.

Ein adaptiver Spatel, wie er hier beschrieben ist, umfasst insbesondere ferner korrespondierende Anschlagflächen an einem vorbestimmten Kettenglied und an dem Abstandsbauteil, wobei die korrespondierenden Anschlagflächen so angeordnet sind, dass ein mechanischer Kontakt der korrespondierenden Anschlagflächen eine Schwenkbewegung des vorbestimmten Kettenglieds relativ zu dem Abstandsbauteil begrenzt.

Das vorbestimmte Kettenglied ist entweder ein Kettenglied der ersten Kette oder ein Kettenglied der zweiten Kette. Das vorbestimmte Kettenglied ist insbesondere ein Kettenglied, das mit dem Abstandsbauteil unmittelbar gelenkig verbunden ist.

Korrespondierende Anschlagflächen, die eine Schwenkbewegung begrenzen, können die Verwendung des adaptiven Spatels vereinfachen oder sicherer machen, insbesondere während des Einführens in den Rachen eines Patienten. Ferner kann eine Begrenzung von Schwenkbewegungen durch korrespondierende Anschlagflächen einer mechanischen Überlastung des adaptiven Spatels, wie sie insbesondere anderenfalls bei einer extremen Konfiguration des adaptiven Spatels auftreten könnte, vorbeugen.

Ein adaptiver Spatel, wie er hier beschrieben ist, umfasst insbesondere ferner ein elastisches Mantelbauteil zum Schutz des adaptiven Spatels vor Verschmutzung und anderen Umwelteinflüssen.

Ein adaptiver Spatel, wie er hier beschrieben ist, umfasst insbesondere ferner einen Kanal, in den zumindest entweder ein Endoskop oder eine Lichtquelle oder ein anderes medizinisches Instrument eingeführt werden oder angeordnet sein kann.

Der Kanal kann aus einer Mehrzahl oder einer Vielzahl von Segmenten, die jeweils ringförmig oder rohrförmig sein und/oder einen U-förmigen Querschnitt aufweisen können, bestehen. Dabei ist insbesondere jedes Segment des Kanals an einem Kettenglied angeordnet oder in ein Kettenglied integriert. Alternativ oder zusätzlich bilden Abstandsbauteile des adaptiven Spatels eine einem Geländer ähnelnde Begrenzung des Kanals.

Ein adaptiver Spatel, wie er hier beschrieben ist, umfasst insbesondere ferner ein Griffbauteil zum Halten und Führen des adaptiven Spatels.

Ein adaptiver Spatel, wie er hier beschrieben ist, kann für eine mehrmalige Verwendung und mehrmalige Sterilisation (insb. Dampfsterilisation im Autoklaven) oder für eine einmalige Verwendung und nachfolgende Entsorgung vorgesehen und ausgebildet sein.

Ein adaptives Laryngoskop oder eine adaptive Intubationsvorrichtung umfasst einen adaptiven Spatel, wie er hier beschrieben ist, und ein Griffbauteil, das mit dem proximalen Ende des adaptiven Spatels mechanisch verbindbar oder verbunden ist.

Das adaptive Laryngoskop ist insbesondere ein Intubationslaryngoskop und/oder für eine Verwendung in der Mikrolarynxchirurgie oder für andere Anwendungen in der Oto-Rhino-Laryngologie vorgesehen.

### Kurzbeschreibung der Figuren

Nachfolgend werden Ausführungsformen anhand der beigefügten Figuren näher erläutert. Es zeigen:
- Figur 1: eine schematische axonometrische Darstellung eines adaptiven Laryngoskops;
- Figur 2: eine schematische Darstellung eines adaptiven Spatels für ein Laryngoskop;
- Figur 3: eine weitere schematische Darstellung des adaptiven Spatels aus Figur 2;
- Figur 4: eine schematische Darstellung eines Teils eines adaptiven Spatels;
- Figur 5: eine schematische Darstellung eines Schnitts durch den Teil des adaptiven Spatels aus Figur 4;
- Figur 6: eine schematische Darstellung eines Schnitts durch einen Teil eines weiteren adaptiven Spatels;
- Figur 7: eine schematische Darstellung eines Schnitts durch einen Teil eines weiteren adaptiven Spatels;
- Figur 8: eine schematische Darstellung eines Teils eines weiteren adaptiven Spatels;
- Figur 9: eine schematische Darstellung eines Schnitts durch den Teil des adaptiven Spatels aus Figur 8;
- Figur 10: eine schematische Darstellung eines Schnitts durch einen weiteren Teil eines adaptiven Spatels;
- Figur 11: eine schematische Darstellung eines Schnitts durch einen weiteren Teil eines weiteren adaptiven Spatels.

### Beschreibung der Ausführungsformen

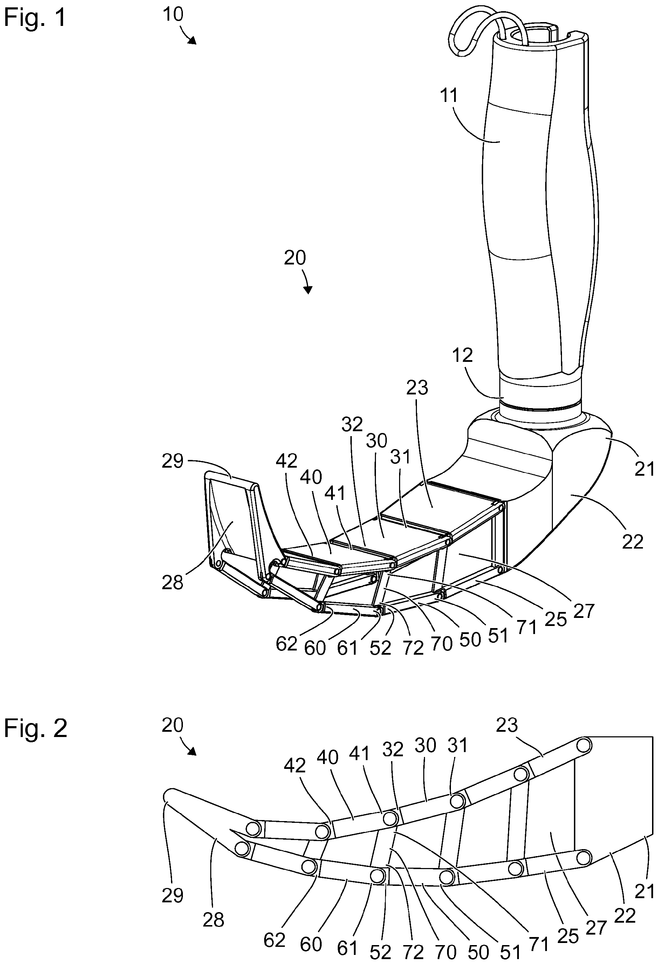

Figur 1 zeigt eine schematische axonometrische Darstellung eines adaptiven Intubationslaryngoskops 10 mit einem Griffbauteil 11 zum manuellen Halten und Führen des Intubationslaryngoskops 10. Ferner umfasst das adaptive Intubationslaryngoskop 10 einen adaptiven Spatel 20 mit einem proximalen Ende 21 und einem distalen Ende 29. Der adaptive Spatel 20 weist einen zum distalen Ende 29 hin sich vermindernden Querschnitt auf. Das proximale Ende 21 des adaptiven Spatels 20 ist derart mit dem Griffbauteil 11 mechanisch verbunden, dass der an das proximale Ende angrenzende Bereich des adaptiven Spatels 20 mit dem Griffbauteil 11 einen Winkel von näherungsweise 90 Grad (im Bereich zwischen ca. 80 Grad und 120 Grad) einschließt.

Der adaptive Spatel umfasst ein proximales Endstück 22, das das proximale Ende 21 des adaptiven Spatels 20 bildet. Ferner umfasst der adaptive Spatel ein distales Endstück 28, das das distale Ende 29 des adaptiven Spatels 20 bildet. Ferner umfasst der adaptive Spatel 20 eine erste Kette 23 und zwei zweiten Ketten 25, die sich von dem proximalen Endstück 22 bis zu dem distalen Endstück 28 des adaptiven Spatels 20 erstrecken.

Die erste Kette 23 umfasst mehrere Kettenglieder 30, 40. Die Kettenglieder 30, 40 der ersten Kette 23 weisen bei dem dargestellten Beispiel leicht unterschiedliche Abmessungen auf, ähneln einander jedoch im Übrigen weitgehend. Entsprechend sind beispielhaft nur zwei Kettenglieder 30, 40 der ersten Kette 23 mit Bezugszeichen versehen.

Die Kettenglieder 30, 40 der ersten Kette 23 sind untereinander durch formschlüssige Gelenke verbunden, so dass das distale Ende 32 eines Kettenglieds 30 jeweils mit dem proximalen Ende 41 des sich distal anschließenden Kettenglieds 40 gelenkig verbunden ist. Das äußerst proximale Kettenglied der ersten Kette 23 ist ferner durch ein formschlüssiges Gelenk mit dem proximalen Endstück 22 des adaptiven Spatels 20 verbunden. Das äußerst distale Kettenglied der ersten Kette 23 ist ferner durch ein formschlüssiges Gelenk mit dem distalen Endstück 28 des adaptiven Spatels 20 verbunden.

Die Kettenglieder 30, 40 der ersten Kette 23 sind jeweils näherungsweise oder im Wesentlichen plattenförmig. Bei dem dargestellten Beispiel weist jedes Kettenglied 30, 40 einen im Wesentlichen trapezförmigen Grundriss auf. Jedes Kettenglied 30, 40 weist eine von proximal nach distal abnehmende Breite auf. Die Breite jedes Kettenglieds 30 an seinem distalen Rand bzw. Ende 32 entspricht der Breite des distal benachbarten Kettenglieds 40 an dessen proximalem Ende 41. Somit nimmt die Breite der ersten Kette 23 von proximal nach distal ab.

Die Breiten der proximalen und distalen Endstücke 22, 28 des adaptiven Spatels 20 sind an die Breiten der ersten Ketten 23 an ihrem proximalen bzw. distalem Ende angeglichen. Entsprechend weist das distale Endstück 28 eine geringere Breite auf als das proximale Endstück 22 des adaptiven Spatels 20.

Jede der beiden zweiten Ketten 25 weist mehrere Kettenglieder 50, 60 auf, die untereinander paarweise gelenkig verbunden sind. Die Kettenglieder 50, 60 der zweiten Ketten 25 gleichen einander oder ähneln einander weitgehend. Alle Kettenglieder 50, 60 einer zweiten Kette 25 oder alle Kettenglieder 50, 60 beider zweiter Ketten 25 können untereinander gleich sein. Einander entsprechende bzw. einander gegenüberliegende Kettenglieder 50, 60 der zweiten Ketten 25 sind insbesondere gleich oder spiegelsymmetrisch zueinander. Entsprechend sind in Fig. 1 nur zwei der Kettenglieder der zweiten Ketten 25 beispielhaft mit Bezugszeichen versehen.

Die Kettenglieder 50, 60 der zweiten Kette 25 sind untereinander paarweise durch formschlüssige Gelenke verbunden, so dass das distale Ende 52 eines Kettenglieds 50 jeweils mit dem proximalen Ende 61 des sich distal anschließenden Kettenglieds 60 gelenkig verbunden ist. Die proximalen Enden der äußerst proximalen Kettenglieder der zweiten Ketten 25 sind jeweils durch ein formschlüssiges Gelenk mit dem proximalen Endstück 22 des adaptiven Spatels 20 verbunden. Die distalen Enden der äußerst distalen Kettenglieder der zweiten Ketten 25 sind jeweils durch ein formschlüssiges Gelenk mit dem distalen Endstück 28 des adaptiven Spatels 20 verbunden.

Der adaptive Spatel 20 umfasst ferner mehrere Abstandsbauteile 70. Jedes Abstandsbauteil 70 weist ein erstes Ende 71 und ein zweites Ende 72 auf. Die Abstandsbauteile 70 ähneln einander weitgehend und unterscheiden sich voneinander insbesondere durch ihre Längen. Deshalb ist nur ein Abstandsbauteil 70 beispielhaft mit Bezugszeichen versehen.

Das erste Ende 71 jedes Abstandsbauteils 70 ist durch ein formschlüssiges Gelenk mit zwei benachbarten Kettengliedern 30, 40 der ersten Kette 23 verbunden. Das zweite Ende 72 jedes Abstandsbauteils 70 ist durch ein formschlüssiges Gelenk mit zwei benachbarten Kettengliedern 50, 60 von einer der beiden zweiten Ketten 25 verbunden.

Die ersten Enden 71 der Abstandsbauteile 70 sind jeweils nahe an den äußeren Rändern der ersten Kette 23 angeordnet. Jeweils zwei Abstandsbauteile 70 sind spiegelsymmetrisch zueinander und parallel oder im Wesentlichen parallel zueinander angeordnet. An jedem Paar von parallelen Abstandsbauteilen 70 entspricht deshalb die Breite der Anordnung der zwei zweiten Ketten 25 im Wesentlichen der Breite der ersten Kette 23 an dem selben Paar von Abstandsbauteilen 70.

Jedes Abstandsbauteil 70 definiert spielarm den Abstand des distalen Endes 32 eines ersten Kettenglieds 30 der ersten Kette 23 und des proximalen Endes 41 eines zweiten Kettenglieds 40 der ersten Kette 23 einerseits von dem distalen Ende 52 eines ersten Kettenglieds 50 der zweiten Kette 25 und dem proximalen Ende 61 eines zweiten Kettenglieds 60 der zweiten Kette 25 andererseits. Da die Längen der Abstandsbauteile 70 von proximal nach distal abnehmen, nehmen auch die Abstände zwischen der ersten Kette 23 und den zweiten Ketten 25 von proximal nach distal ab. Dies bewirkt, wie in Figur 1 angedeutet, dass eine Bewegung der Ketten 23, 25 in einem mittleren Bereich in eine Richtung (in Figur 1: nach unten) mit einer Schwenkbewegung des distalen Endes 29 des adaptiven Spatels 20 in entgegengesetzter Richtung (in Figur 1: nach oben) einhergeht. Dies kann eine Anformung des adaptiven Spatels 20 an eine Zunge eines Patienten und eine Verteilung einer auf die Zunge eines Patienten ausgeübten Kraft ermöglichen.

Die Kettenglieder 30, 40 der ersten Kette 23, die Kettenglieder 50, 60 der zweiten Kette 25 und die Abstandsbauteile 70 weisen zusammen eine Gestalt mit im Wesentlichen rechteckig U-förmigem Querschnitt auf, wobei die Abstandsbauteile 70 und die zweiten Ketten 25 keine geschlossenen Seitenwände, sondern lediglich Zäunen ähnliche Strukturen bilden. Ein Raumbereich innerhalb des U-förmigen Querschnitts bildet einen Kanal 27, durch den eine Lichtquelle, eine Kamera, ein Endoskop oder ein anderes medizinisches Instrument teilweise oder vollständig hindurchgeführt oder in dem eine Lichtquelle, eine Kamera, ein Endoskop oder ein anderes medizinisches Instrument angeordnet werden kann.

Figur 2 zeigt eine schematische Darstellung eines adaptiven Spatels 20, der in einigen Merkmalen, Eigenschaften und Funktionen dem adaptiven Spatel 20 des anhand der Figur 1 dargestellten Laryngoskops ähnelt. Der in Figur 2 dargestellte Spatel 20 ist insbesondere zur lösbaren oder dauerhaften mechanischen Verbindung mit einem Griffbauteil vorgesehen und ausgebildet, um ein Laryngoskop zu bilden.

In Figur 2 deuten kleine Kreise die formschlüssigen Gelenke zwischen den Kettengliedern 30, 40 der ersten Kette 23, den Enden 71, 72 der Abstandsbauteile 70 und den Kettengliedern 50, 60 der zweiten Kette 25 an. Die Schwenkachsen und Symmetrieachsen 88, 98 aller formschlüssiger Gelenke sind orthogonal zu der Zeichenebene der Figur 2.

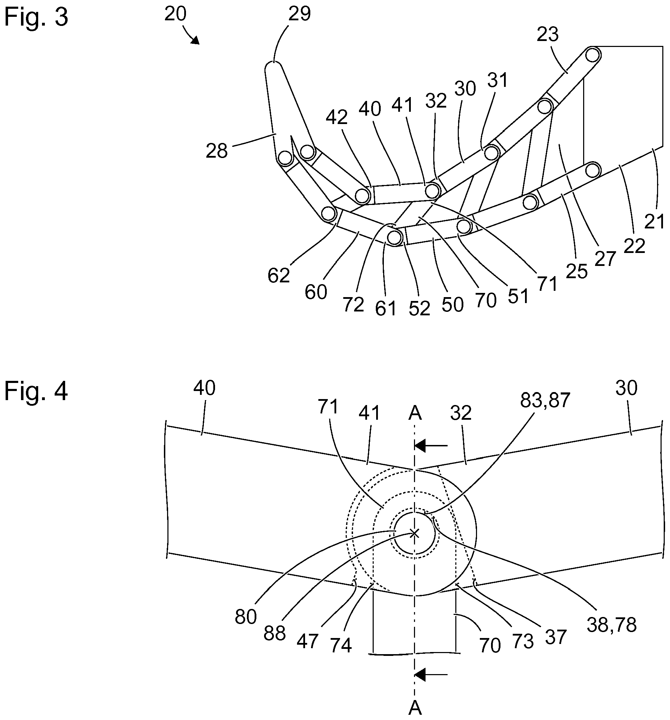

Figur 3 zeigt eine weitere schematische Darstellung des adaptiven Spatels 20 aus Figur 2. Die Zeichenebene der Figur 3 entspricht der Zeichenebene der Figur 2.

In Figur 3 ist der adaptive Spatel 20 in einer Konfiguration bzw. Situation gezeigt, die sich von der in Figur 2 gezeigten Situation unterscheidet. Die in Figur 3 gezeigte Konfiguration entsteht beispielsweise durch Anformung des adaptiven Spatels 20 an die gewölbte Oberfläche der Zunge eines Patienten. Dabei weichen Kettenglieder 30, 40, 50, 60 in einem mittleren Bereich der Ketten 23, 25 in eine Richtung (in Figur 3: nach unten) aus, während das distale Endstück 28 des adaptiven Spatels 20 in die Gegenrichtung (in Figur 3: nach oben) geschwenkt wird.

Figur 4 zeigt eine schematische Darstellung eines Teils eines adaptiven Spatels, wie er anhand der Figuren 1 bis 3 beschrieben ist. Die Zeichenebene der Figur 4 ist parallel zu den Zeichenebenen der Figuren 2 und 3.

In Figur 4 ist die gelenkige Verbindung zwischen dem distalen Ende 32 eines ersten Kettenglieds 30, dem proximalen Ende 41 eines zweiten Kettenglieds 40 und einem ersten Ende 71 eines Abstandsbauteils 70 dargestellt. Das zweite Kettenglied 40 verdeckt das erste Kettenglied 30 und das Abstandsbauteil 70 teilweise. Konturen des ersten Kettenglieds 30 und des Abstandsbauteils 70 sind, soweit sie verdeckt und deshalb eigentlich nicht sichtbar sind, durch gestrichelte Linien angedeutet. Ferner sind weitere in der Ansicht der Figur 4 eigentlich nicht sichtbare Strukturen durch gestrichelte Linien angedeutet.

Die gelenkige Verbindung der Kettenglieder 30, 40 und des Abstandsbauteils 70 erfolgt durch eine Welle 80 mit kreiszylindrischem Querschnitt. Die Welle 80 weist eine Symmetrieachse 88 auf, die die Schwenkachse der gelenkigen Verbindung bildet. Das dem Betrachter zugewandte Ende der Welle 80 ist in eine entsprechende Bohrung in dem zweiten Kettenglied 40 eingesetzt und mit diesem reib- und/oder stoffschlüssig gefügt. Ringförmige Bereiche der Oberfläche der Welle 80 bilden Lagerflächen 83, 87. An dem ersten Kettenglied 30 ist eine zu der Lagerfläche 83 korrespondierende, insbesondere ihr gegenüberliegende Lagerfläche 38 vorgesehen. An dem Abstandsbauteil 70 ist eine zu der Lagerfläche 87 an der Welle 80 korrespondierende, insbesondere ihr gegenüberliegende Lagerfläche 78 vorgesehen. In Figur 4 ist ein Abstand zwischen korrespondierenden Lagerflächen 83, 38 und 87, 78 angedeutet, der reibungsarm Bewegungen zulässt. Abweichend von der Darstellung in Figur 4 können Wälzkörper zwischen den korrespondierenden Lagerflächen 83, 38 und/oder zwischen den korrespondierenden Lagerflächen 87, 78 vorgesehen sein.

An dem ersten Kettenglied 30 ist eine Anschlagfläche 37 vorgesehen. An dem Abstandsbauteil 70 ist eine zu der Anschlagfläche 37 an dem ersten Kettenglied 30 korrespondierende erste Anschlagfläche 73 vorgesehen. Ein mechanischer Kontakt zwischen den korrespondierenden Anschlagflächen 37, 73 an dem ersten Kettenglied 30 und an dem Abstandsbauteil 70 begrenzt eine Schwenkbewegung des Abstandsbauteils 70 relativ zu dem ersten Kettenglied 30.

An dem zweiten Kettenglied 40 ist eine Anschlagfläche 47 vorgesehen. An dem Abstandsbauteil 70 ist eine zu der Anschlagfläche 47 an dem zweiten Kettenglied 40 korrespondierende zweite Anschlagfläche 74 vorgesehen. Ein mechanischer Kontakt zwischen den Anschlagflächen 47, 74 an dem zweiten Kettenglied 40 und an dem Abstandsbauteil 70 begrenzt eine Schwenkbewegung des zweiten Kettenglieds 40 relativ zu dem Abstandsbauteil 70.

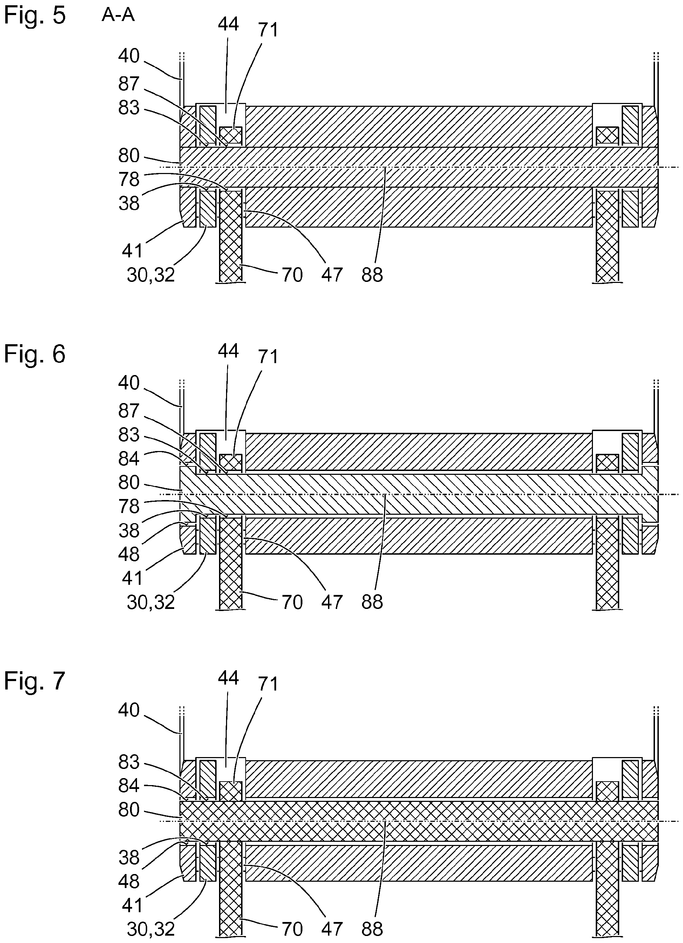

Figur 5 zeigt eine schematische Darstellung eines Schnitts entlang der in Figur 4 angedeuteten Schnittebene A-A durch die in Figur 4 gezeigte gelenkige Verbindung der Kettenglieder 30, 40 und des Abstandsbauteils 70. Die Schnittebene A-A der Figur 5 ist orthogonal zu den Zeichenebene der Figur 4 und enthält die Schwenkachse und Symmetrieachse 88 der ersten Welle 80.

Die Schnittebene A-A der Figur 5 schneidet das distale Ende 32 des ersten Kettenglieds 30, das proximale Ende 41 des zweiten Kettenglieds 40 und zwei Abstandsbauteile 70. Die Kettenglieder 30, 40 sind spiegelsymmetrisch zu einer Symmetrieebene, die orthogonal zu der Schnittebene A-A der Figur 5 und zu der Symmetrieachse 88 der Welle 80 ist, ausgebildet. Die Abstandsbauteile 70 sind spiegelsymmetrisch zu derselben Symmetrieebene angeordnet. Deshalb sind Bezugszeichen nur an einer (in Figur 5 links dargestellten) Seite vorgesehen.

Das proximale Ende 41 des zweiten Kettenglieds 40 weist zwei Ausnehmungen 44 auf. Das distale Ende 32 des ersten Kettenglieds 30 wird durch zwei zungenförmige oder lamellenartige Fortsätze gebildet, die in die Ausnehmungen 44 an dem proximalen Ende 41 des zweiten Kettenglieds 40 eingreifen. In jede Ausnehmung 44 in dem proximalen Ende 41 des zweiten Kettenglieds 40 greift ferner ein Ende eines Abstandsbauteils 70 ein.

Die Welle 80 ist spielarm und insbesondere reibschlüssig in eine korrespondierende Bohrung in dem proximalen Ende 41 des zweiten Kettenglieds 40 eingesetzt. Die Ausnehmungen 44 in dem proximalen Ende 41 des zweiten Kettenglieds 40 sind so angeordnet, dass die Welle 80 beide Ausnehmungen 44 durchdringt. In den Ausnehmungen 44 liegen Teilbereiche der äußeren Oberfläche der Welle 80 offen, die die Lagerflächen 83, 87 bilden. Die das distale Ende 32 des ersten Kettenglieds 30 bildenden Zungen oder Lamellen weisen je eine Bohrung auf, durch die hindurch die Welle 80 greift, und deren innere Oberflächen zu den Lagerflächen 83 an der Welle 80 korrespondierende Lagerflächen 38 bilden. Die Enden 71 der Abstandsbauteile 70 weisen je eine Bohrung auf, durch die hindurch die Welle 80 greift, und deren innere Oberflächen zu den Lagerflächen 87 an der Welle 80 korrespondierende Lagerflächen 78 bilden.

Durch gleiche Schraffuren ist angedeutet, dass bei dem in den Figuren 4 und 5 gezeigten Beispiel die Welle 80 starr mit dem proximalen Ende 41 des zweiten Kettenglieds 40 verbunden ist und insofern einen Teil des zweiten Kettenglieds 40 bildet. Die Lagerflächen 38 an dem distalen Ende 32 des ersten Kettenglieds 30 und die dazu korrespondierenden Lagerflächen 83 an der Welle 80 bilden ein formschlüssiges Gelenk zwischen dem ersten Kettenglied 30 und dem zweiten Kettenglied 40. Die Lagerflächen 78 an den Abstandsbauteilen 70 und die dazu korrespondierenden Lagerflächen 87 an der Welle 80 bilden formschlüssige Gelenke zwischen dem zweiten Kettenglied 40 und den Abstandsbauteilen 70.

Die Bereiche der Welle 80 innerhalb der Ausnehmungen 44 in dem proximalen Ende 41 des zweiten Kettenglieds 40, die daran befindlichen Lagerflächen 83, 87 und die korrespondierenden Lagerflächen 38, 78 an dem distalen Ende 32 des ersten Kettenglieds 30 und an dem Ende 71 des Abstandsbauteils 70 bilden eine Mehrfachgelenkeinrichtung. Diese Mehrfachgelenkeinrichtung vereint auf kleinem Bauraum formschlüssige Gelenke zwischen beiden Kettengliedern 30, 40 und dem Abstandsbauteil 70.

Figur 6 zeigt eine schematische Darstellung eines Schnitts durch eine alternative Ausgestaltung einer gelenkigen Verbindung zwischen Kettengliedern 30, 40 und einem Abstandsbauteil 70 eines adaptiven Spatels, wie er anhand der Figuren 1 bis 3 dargestellt ist. Die Art der Darstellung, insbesondere die Schnittebene, entspricht derjenigen der Figur 5. Die in Figur 6 dargestellte Ausgestaltung ähnelt in einigen Merkmalen, Eigenschaften und Funktionen der anhand der Figuren 4 und 5 dargestellten Ausgestaltung. Nachfolgend sind insbesondere Merkmale, Eigenschaften und Funktionen beschrieben, in denen die in Figur 6 gezeigte Ausgestaltung sich von der anhand der Figuren 4 und 5 dargestellten Ausgestaltung unterscheidet.

Bei der in Figur 6 gezeigten gelenkigen Verbindung ist die Welle 80 nicht starr mit dem zweiten Kettenglied 40 verbunden. Vielmehr sind die Welle 80 und die korrespondierende Bohrung in dem proximalen Ende 41 des zweiten Kettenglieds 40 so ausgestaltet, dass die Welle 80 nicht reibschlüssig gehalten ist, sondern Lagerflächen 48 bilden. An der Welle 80 sind zu den Lagerflächen 48 an dem proximalen Ende 41 des zweiten Kettenglieds 40 korrespondierende Lagerflächen 84 gebildet. In Figur 6 deutet ein Lagerspiel zwischen den korrespondierenden Lagerflächen 48, 84 eine Rotierbarkeit der Welle 80 relativ zu dem zweiten Kettenglied 40 an. Die Welle 80 kann somit sowohl relativ zu dem Abstandsbauteil 70 und dem distalen Ende 32 des ersten Kettenglieds 30 als auch relativ zu dem proximalen Ende 41 des zweiten Kettenglieds 40 rotieren.

Somit wird ein formschlüssiges Gelenk zwischen den Kettengliedern 30, 40 durch die Lagerflächen 38 an dem distalen Ende 32 des ersten Kettenglieds 30, die dazu korrespondierenden Lagerflächen 83 an der Welle 80, die Lagerflächen 48 an dem proximalen Ende 41 des zweiten Kettenglieds 40 und die dazu korrespondierenden Lagerflächen 84 an der Welle 80 gebildet. Entsprechend werden auch alle übrigen gelenkigen Verbindungen über die Welle 80 gebildet.

Um ein Herausgleiten der Welle 80 aus den Bohrungen in den Kettengliedern 30, 40 und in den Abstandsbauteilen 70 durch eine Bewegung parallel zu der Symmetrieachse 88 der Welle 80 zu unterbinden, weisen bei dem dargestellten Beispiel die Enden der Welle 80 jeweils einen vergrößerten Durchmesser auf.

Figur 7 zeigt eine schematische Darstellung eines Schnitts durch eine weitere Ausgestaltung einer gelenkigen Verbindung zwischen Kettengliedern 30, 40 und Abstandsbauteilen 70, die in einigen Merkmalen, Eigenschaften und Funktionen den anhand der Figuren 4 bis 6 dargestellten gelenkigen Verbindungen ähnelt. Die Art der Darstellung, insbesondere die Schnittebene, entspricht derjenigen der Figuren 5 und 6. Nachfolgend sind Merkmale, Eigenschaften und Funktionen beschrieben, in denen die in Figur 7 gezeigte Ausgestaltung sich von den anhand der Figuren 4 bis 6 dargestellten Ausgestaltungen unterscheidet.

Bei der in Figur 7 gezeigten gelenkigen Verbindung ist die Welle 80 reib- bzw. kraftschlüssig und/oder stoffschlüssig mit den Abstandsbauteilen 70 verbunden. Die Welle 80 ist insofern Bestandteil der Abstandsbauteile 70 und verbindet die Abstandsbauteile miteinander starr. Dies ist durch die Schraffur der Schnittflächen in Figur 7 angedeutet. Die starre Verbindung der Abstandsbauteile 70 kann die Steifheit des gesamten adaptiven Spatels erhöhen.

Bei der in Figur 7 gezeigten gelenkigen Verbindung stellen die Lagerfläche 38 an dem distalen Ende 32 des ersten Kettenglieds 30 und die korrespondierenden Lagerflächen 83 an der Welle 80 formschlüssige Gelenke zwischen dem ersten Kettenglied 30 und den Abstandsbauteilen 70 dar. Lagerflächen 48 an dem proximalen Ende 41 des zweiten Kettenglieds 40 und korrespondierende Lagerflächen 84 an der Welle 80 bilden formschlüssige Gelenke zwischen dem proximalen Ende 41 des zweiten Kettenglieds 40 und den Abstandsbauteilen 70. Die Lagerflächen 83, 84 an der Welle 80 und die korrespondierenden Lagerflächen 38, 48 an den Kettengliedern 30, 40 bilden zusammen Mehrfachgelenkeinrichtungen, die gleichzeitig gelenkige Verbindungen zwischen den Kettengliedern 30, 40 bildet.

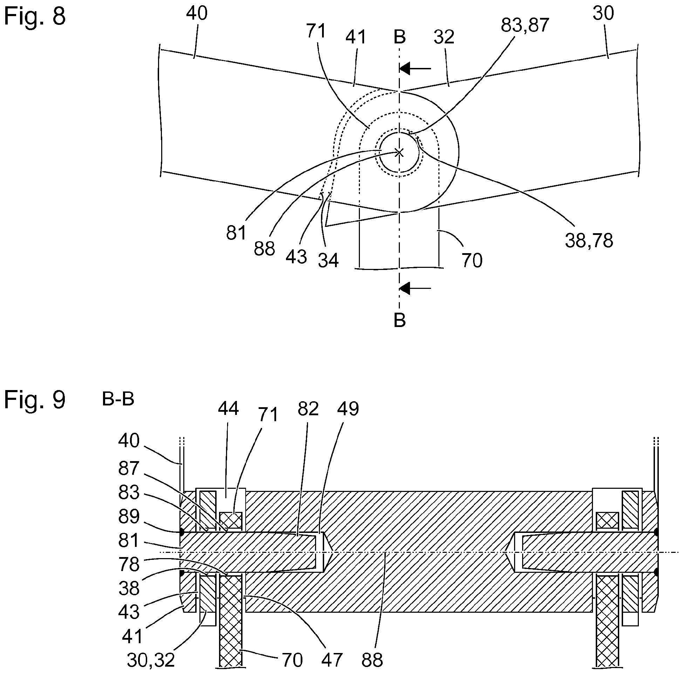

Figur 8 zeigt eine schematische Darstellung einer alternative Ausgestaltung einer gelenkigen Verbindung zwischen Kettengliedern 30, 40 und einem Abstandsbauteil 70 eines adaptiven Spatels, wie er anhand der Figuren 1 bis 3 dargestellt ist. Die Art der Darstellung in Figur 8 entspricht derjenigen der Figur 4. Die in Figur 8 dargestellte Ausgestaltung ähnelt in einigen Merkmalen, Eigenschaften und Funktionen den anhand der Figuren 4 bis 7 dargestellten Ausgestaltungen. Nachfolgend sind insbesondere Merkmale, Eigenschaften und Funktionen beschrieben, in denen die in Figur 8 gezeigte Ausgestaltung sich von der anhand den Figuren 4 bis 7 dargestellten Ausgestaltungen unterscheidet.

Die in Figur 8 gezeigte Ausgestaltung unterscheidet sich von den anhand der Figuren 4 und 5 dargestellten Ausgestaltung insbesondere dadurch, dass eine Anschlagfläche 34 an dem distalen Ende 32 des ersten Kettenglieds 30 und eine dazu korrespondierende Anschlagfläche 43 an dem proximalen Ende 41 des zweiten Kettenglieds 40 vorgesehen sind. Ein mechanischer Kontakt zwischen den korrespondierenden Anschlagflächen 34, 43 an dem ersten Kettenglied 30 und an dem zweiten Kettenglied begrenzt eine Schwenkbewegung der Kettenglieder 30, 40 relativ zueinander.

Bei der in Figur 8 gezeigten Ausgestaltung ist keine Anschlagfläche an dem Abstandsbauteil 70 vorgesehen. Alternativ und abweichend von der Darstellung in Figur 8 können zusätzlich zu den Anschlagflächen 34, 43 weitere Anschlagflächen vorgesehen sein, deren mechanischer Kontakt eine Bewegung des Abstandsbauteils 70 relativ zu einem oder beiden Kettengliedern 30, 40 begrenzt. Anders ausgedrückt können an einer Mehrfachgelenkeinrichtung gleichzeitig die anhand der Figur 4 dargestellten Anschlagflächen 47, 74 zur unmittelbaren Begrenzung einer Relativbewegung von Abstandsbauteil 70 und Kettengliedern 30, 40 und die anhand der Figur 8 dargestellten Anschlagflächen 34, 43 zur unmittelbaren Begrenzung einer Relativbewegung der Kettenglieder 30, 40 vorgesehen sein.

Figur 9 zeigt eine schematische Darstellung eines Schnitts entlang der in Figur 8 angedeuteten Schnittebene B-B durch die in Figur 8 gezeigte gelenkige Verbindung der Kettenglieder 30, 40 und des Abstandsbauteils 70. Die Schnittebene B-B der Figur 9 ist orthogonal zu den Zeichenebene der Figur 8 und enthält die Schwenkachse 88.

In Figur 8 ist erkennbar, dass die darin gezeigte Ausgestaltung sich von den anhand der Figuren 4 und 5 dargestellten Ausgestaltung ferner dadurch unterscheidet, dass anstelle einer durchgehenden, sich über die gesamte Breite des zweiten Kettenglieds 40 erstreckenden Welle zwei kurze Wellen 81 vorgesehen sind. Jede der kurzen Wellen 81 weist die Gestalt eines Stifts mit einem konischen Bereich 82 auf. Die Schwenkachse 88 ist gleichzeitig Symmetrieachsen der Wellen 81.

Das zweite Kettenglied 40 weist zwei spiegelsymmetrisch angeordnete und von einander abgewandte Sackbohrungen 49 auf. Jede der kurzen Wellen 81 ist so in eine der beiden Sackbohrung 49 eingesetzt, dass der konische Bereich 82 der Welle 81 dem geschlossenen Ende der Sackbohrung 49 zugewandt ist. Die konischen Bereiche 82 der Wellen 81 vereinfacht bei der Montage des adaptiven Spatels das Einsetzen der Wellen 81 in die Sackbohrungen 49.

Ähnlich wie bei den anhand der Figuren 4 bis 7 dargestellten Ausgestaltungen greifen die Wellen 81 durch die Ausnehmungen 44 in dem proximalen Ende 41 des zweiten Kettenglieds hindurch. Oberflächenbereiche der Wellen 81 innerhalb der Ausnehmungen 44 bilden Lagerflächen 83, 87 mit den beschriebenen Funktionen. Jede Ausnehmung 44 ist zwischen dem offenen Ende und dem geschlossenen Ende der zugeordneten Sackbohrung 49 und zwischen den Enden der zugeordneten Welle 81 angeordnet.

Jede Welle 81 ist durch eine Übermaßpassung kraft- bzw. reibschlüssig in der zugeordneten Sackbohrung 49 gehalten. Die Übermaßpassung umfasst einen Abschnitt oder Bereich der Sackbohrung 49, in dem der Innendurchmesser der Sackbohrung 49 vor dem Einsetzen der Welle 81 (d. h. im mechanisch spannungsfreien Zustand) kleiner ist als der Außendurchmesser der Welle 81 in dem dazu korrespondierenden Bereich vor dem Einsetzen in die Sackbohrung 49 (d. h. im mechanisch spannungsfreien Zustand). Dies führt beim Einsetzen der Wellen 81 in die Sackbohrungen 49 zu Verformungen und mechanischen Spannungen in den Wellen 81 und in den Umgebungen der Sackbohrungen 49. Die resultierenden Rückstellkräfte halten die Wellen 81 kraft- bzw. reibschlüssig in den Sackbohrungen 49. Diese Übermaßpassung ist jeweils in einem Bereich zwischen der Ausnehmung 44 und dem geschlossenen Ende der Sackbohrung 49 lokalisiert.

Zusätzlich ist jede Welle 81 durch eine kreisförmige Schweißnaht 89 mit dem proximalen Ende 41 des zweiten Kettenglieds 40 stoffschlüssig verbunden. Die kreisförmigen Schweißnähte 89 sind an den offenen Enden der Sackbohrungen 49 vorgesehen. Die Enden der Wellen 81 an den Schweißnähten 89, die umgebenden Oberflächenbereiche des zweiten Kettenglieds 40 und die Schweißnähte 89 sind insbesondere eben bzw. plan geschliffen und/oder poliert.

Jede Welle 81 kann alternativ und abweichend von der Darstellung anhand der Figuren 8 und 9 nur entweder durch eine Übermaßpassung oder durch eine Schweißnaht 89 mit dem zweiten Kettenglied 40 gefügt sein. Jede Welle 81 kann ferner abweichend von der Darstellung anhand der Figuren 8 und 9 alternativ oder zusätzlich zu der Schweißnaht 89 und/oder der Übermaßpassung durch Schraubgewinde oder auf andere Weise form-, stoff- oder kraft- bzw. reibschlüssig mit dem zweiten Kettenglied 40 oder mit dem ersten Kettenglied 30 mechanisch verbunden sein.

Abweichend von der Darstellung in den Figuren 8 und 9 kann bei einer Ausgestaltung mit den in Figur 8 gezeigten Anschlagflächen 34, 43 zur Begrenzung der Relativbewegung der Kettenglieder 30, 40 anstelle zweier kurzer Wellen 81 eine durchgehende Welle vorgesehen sein, wie sie anhand der Figuren 4 bis 7 beschrieben ist.

Abweichend von der Darstellung in den Figuren 8 und 9 können bei einer Ausgestaltung mit den in Figur 8 gezeigten zwei kurzen Wellen 81 anstelle der Anschlagflächen 34, 43 (zur Begrenzung der Relativbewegung der Kettenglieder 30, 40) oder zusätzlich Anschlagflächen 37, 73, 47, 74 zur Begrenzung der Bewegung des Abstandsbauteils 70 relativ zu den Kettengliedern 30, 40 vorgesehen sein.

Gelenkige Verbindungen zwischen den Kettengliedern 50, 60 der zweiten Kette 25 und den zweiten Enden 72 der Abstandsbauteile 70 des adaptiven Spatels 20 (vgl. Figuren 1 bis 3) können ähnlich ausgebildet sein, wie die anhand der Figuren 4 bis 9 dargestellten formschlüssigen Gelenke zwischen den Kettengliedern 30, 40 der ersten Ketten 23 und den ersten Enden 71 der Abstandsbauteile 70. Insbesondere sind jedoch an den gelenkigen Verbindungen zwischen den Kettengliedern 50, 60 der zweiten Kette 25 und den zweiten Enden 72 der Abstandsbauteile 70 keine oder keine bei der vorgesehenen Verwendung des adaptiven Spatels 20 wirksame Anschlagflächen vorgesehen. Alternativ können auch an den gelenkigen Verbindungen zwischen den Kettengliedern 50, 60 der zweiten Kette 25 und den zweiten Enden 72 der Abstandsbauteile 70 korrespondierende Paare von Anschlagflächen vorgesehen sein, um Relativbewegungen zu begrenzen.

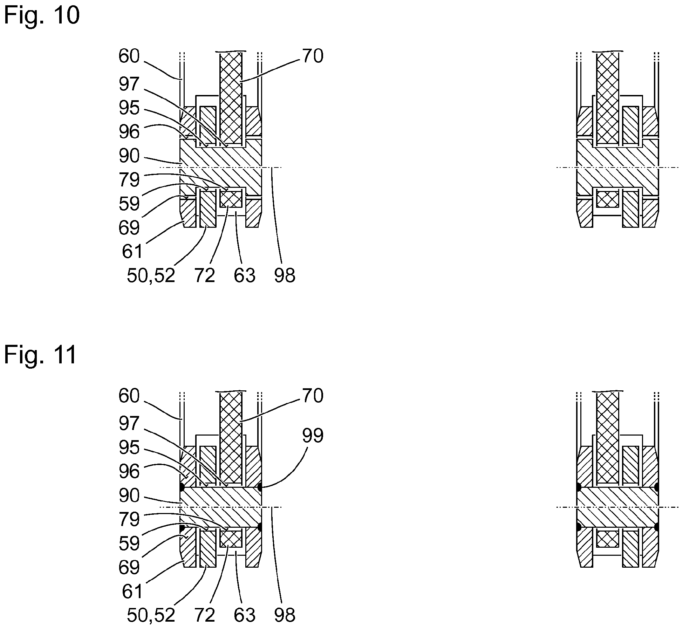

Figur 10 zeigt eine schematische Darstellung eines Schnitts durch gelenkige Verbindungen zwischen Kettengliedern 50, 60 zweiter Ketten 25 und Abstandsbauteilen 70 eines adaptiven Spatels 20 (vgl. Figuren 1 bis 3). Da die durch die Kettenglieder 50, 60 gebildeten zweiten Ketten und die Abstandsbauteile 70 spiegelsymmetrisch angeordnet sind, sind in Figur 10 nur eine (nämlich die linke) Kette und das linke Abstandsbauteil 70 mit Bezugszeichen versehen.

In den proximalen Enden 61 der zweiten Kettenglieder 60 sind Ausnehmungen 63 vorgesehen. In den distalen Enden 52 der ersten Kettenglieder 50 und in den proximalen Enden 61 der zweiten Kettenglieder 60 und in den Enden 72 der Abstandsbauteile 70 sind Bohrungen vorgesehen, in denen zweite Wellen 90 angeordnet sind. Die zweiten Wellen 90 durchdringen die Ausnehmungen 63 in den proximalen Enden 61 der zweiten Kettenglieder 60. Innerhalb der Ausnehmungen 63 liegen die zweiten Wellen 90 frei und durchdringen die Bohrungen in den distalen Enden 52 der ersten Kettenglieder und in den Enden 72 der Abstandsbauteile 70. Innere Oberflächen der Bohrungen bilden Lagerflächen 59, 69, 79, diesen gegenüberliegende Oberflächenbereiche der zweiten Wellen 90 bilden korrespondierende Lagerflächen 95, 96, 97.

Die Lagerfläche 59 an den distalen Enden 52 der ersten Kettenglieder 50, die dazu korrespondierenden Lagerflächen 95 an den zweiten Wellen 90, die Lagerflächen 69 an den proximalen Enden 61 der zweiten Kettenglieder 60 und die dazu korrespondierenden Lagerflächen 96 an den zweiten Wellen 90 bilden formschlüssige Gelenke zwischen den Kettengliedern 50, 60. Die Lagerflächen 59 an den distalen Enden 52 der ersten Kettenglieder 50, die dazu korrespondierenden Lagerflächen 95 an den zweiten Wellen 90, die Lagerflächen 79 an den Abstandsbauteilen 70 und die dazu korrespondierenden Lagerflächen 97 an den zweiten Wellen 90 bilden formschlüssige Gelenke zwischen den ersten Kettengliedern 50 und den Abstandsbauteilen 70. Die Lagerflächen 69 an den proximalen Enden 61 der zweiten Kettenglieder 60, die dazu korrespondierenden Lagerflächen 96 an den zweiten Wellen 90, die Lagerflächen 79 an den Enden 72 der Abstandsbauteilen 70 und die dazu korrespondierenden Lagerflächen 97 an den zweiten Wellen 90 bilden formschlüssige Gelenke zwischen den zweiten Kettengliedern 60 und den Abstandsbauteilen 70.

Abweichend von der Darstellung in Figur 10 können die zweiten Wellen 90 beispielsweise analog zu der anhand der Figuren 4 und 5 dargestellten gelenkigen Verbindung mit den zweiten Kettengliedern 60 oder analog zu der anhand der Figur 7 dargestellten gelenkigen Verbindung starr mit den Abstandsbauteilen 70 oder starr mit den distalen Enden 52 der ersten Kettenglieder 50 verbunden sein.

Figur 11 zeigt eine schematische Darstellung eines Schnitts durch eine alternative Ausgestaltung der gelenkigen Verbindungen zwischen Kettengliedern 50, 60 zweiter Ketten 25 und Abstandsbauteilen 70 eines adaptiven Spatels 20 (vgl. Figuren 1 bis 3), die der anhand der Figur 10 dargestellten Ausgestaltung in einigen Merkmalen, Eigenschaften und Funktionen ähnelt. Die Art der Darstellung, insbesondere die dargestellte Schnittebene, entspricht derjenigen der Figur 10. Nachfolgend sind insbesondere Merkmale, Eigenschaften und Funktionen beschrieben, in denen die in Figur 11 gezeigte Ausgestaltung sich von der anhand der Figur 10 dargestellten Ausgestaltung unterscheidet.

Bei der in Figur 11 gezeigten Ausgestaltung sind die Wellen 90 durch Schweißnähte 99 mit den proximalen Enden 61 der zweiten Kettenglieder 60 gefügt. Die Schweißnähte 99 sind jeweils kreisförmig. An jedem Ende jeder Welle 90 ist eine das Ende umschließende Schweißnaht 99 vorgesehen. Die Enden der Wellen 90 an den Schweißnähten 99, die umgebenden Oberflächenbereiche der zweiten Kettenglieder 60 und die Schweißnähte 99 sind insbesondere eben bzw. plan geschliffen und/oder poliert.

An jeder Welle 90 kann abweichend von der Darstellung anhand der Figur 11 nur eine Schweißnaht 99 vorgesehen sein. Jede Welle 90 kann abweichend von der Darstellung anhand der Figur 11 alternativ oder zusätzlich zu einer oder zu beiden Schweißnähten 99 auf andere Weise mit dem proximalen Ende 61 des zweiten Kettenglieds 60 gefügt sein.

Jede Welle 80, 81, 90 kann abweichend von den Darstellungen anhand der Figuren 4 bis 11 mit dem distalen Ende 32, 52 des ersten Kettenglieds 30, 50 statt mit dem proximalen Ende 41, 61 des distal benachbarten zweiten Kettenglieds gefügt sein.

### Bezugszeichen

- 10: Laryngoskop
- 11: Handgriff des Laryngoskops 10
- 12: distales Ende des Handgriffs 10 des Laryngoskops 10
- 20: adaptiver Spatel des Laryngoskops 10
- 21: proximales Ende des adaptiven Spatels 20
- 22: proximales Endstück des adaptiven Spatels 20
- 23: erste Kette des adaptiven Spatels 20
- 25: zweite Kette des adaptiven Spatels 20
- 27: Kanal für ein medizinisches Instrument an dem adaptiven Spatel 20
- 28: distales Endstück des adaptiven Spatels 20
- 29: distales Ende des adaptiven Spatels 20
- **30**: **erstes Kettenglied der ersten Kette 23** des adaptiven Spatels 20
- 31: proximales Ende des ersten Kettenglieds 30 der ersten Kette 23
- 32: distales Ende des ersten Kettenglieds 30 der ersten Kette 23
- 34: Anschlagfläche an dem ersten Kettenglied 30 der ersten Kette 23, zu der Anschlagfläche 43 an dem zweiten Kettenglied 40 der ersten Kette 23 korrespondierend
- 37: Anschlagfläche an dem ersten Kettenglied 30 der ersten Kette 23, zu der Anschlagfläche 73 an dem Abstandsbauteil 70 korrespondierend
- 38: Lagerfläche an dem ersten Kettenglied 30 der ersten Kette 23, zu der Lagerfläche 83 an der ersten Welle 80 korrespondierend
- **40**: **zweites Kettenglied der ersten Kette 23** des adaptiven Spatels 20
- 41: proximales Ende des zweiten Kettenglied 40 der ersten Kette 23
- 42: distales Ende des zweiten Kettenglieds 40 der ersten Kette 23
- 43: Anschlagfläche an dem zweiten Kettenglied 40 der ersten Kette 23, zu der Anschlagfläche 34 an dem ersten Kettenglied 30 der ersten Kette 23 korrespondierend
- 44: Ausnehmung an dem proximalen Ende 41 des zweiten Kettenglieds 40
- 47: Anschlagfläche an dem zweiten Kettenglied 40 der ersten Kette 23, zu der Anschlagfläche 74 an dem Abstandsbauteil 70 korrespondierend
- 48: Lagerfläche an dem zweiten Kettenglied 40 der ersten Kette 23, zu der Lagerfläche 84 an der ersten Welle 80 korrespondierend
- 49: Sackbohrung in dem zweiten Kettenglied 40
- **50**: **erstes Kettenglied der zweiten Kette 25** des adaptiven Spatels 20
- 51: proximales Ende des ersten Kettenglieds 50 der zweiten Kette 25
- 52: distales Ende des ersten Kettenglieds 50 der zweiten Kette 25
- 59: Lagerfläche an dem ersten Kettenglied 50 der zweiten Kette 25, zu der Lagerfläche 95 an der zweiten Welle 90 korrespondierend
- **60**: **zweites Kettenglied der zweiten Kette 25** des adaptiven Spatels 20
- 61: proximales Ende des zweiten Kettenglieds 60 der zweiten Kette 25
- 62: distales Ende des zweiten Kettenglieds 60 der zweiten Kette 25
- 63: Ausnehmung an dem proximalen Ende 61 des zweiten Kettenglieds 60
- 69: Lagerfläche an dem zweiten Kettenglied 60 der zweiten Kette 25, zu der Lagerfläche 96 an der zweiten Welle 90 korrespondierend
- **70**: **Abstandsbauteil** des adaptiven Spatels 20
- 71: erstes Ende des Abstandsbauteils 70
- 72: zweites Ende des Abstandsbauteils 70
- 73: erste Anschlagfläche an dem Abstandsbauteil 70, zu der Anschlagfläche 37 an dem ersten Kettenglied 30 der ersten Kette 23 korrespondierend
- 74: zweite Anschlagfläche an dem Abstandsbauteil 70, zu der Anschlagfläche 47 an dem zweiten Kettenglied 40 der ersten Kette 23 korrespondierend
- 78: zu der Lagerfläche 87 an der ersten Welle 80 korrespondierende Lagerfläche an dem Abstandsbauteil 70
- 79: zu der Lagerfläche 97 an der zweiten Welle 90 korrespondierende Lagerfläche an dem Abstandsbauteil 70
- **80**: **erste Welle** zur gelenkigen Verbindung zweier Kettenglieder 30, 40 der ersten Kette 23 und des ersten Endes 71 eines Abstandsbauteils 70
- 81: Stift als Welle zur gelenkigen Verbindung zweier Kettenglieder 30, 40 der ersten Kette 23 und des ersten Endes 71 eines Abstandsbauteils 70, in das Sackloch 49 eingesetzt
- 82: konischer Bereich an einem Ende des Stifts 81
- 83: zu der Lagerfläche 38 an dem ersten Kettenglied 30 der ersten Kette 23 korrespondierende Lagerfläche an der ersten Welle 80
- 84: zu der Lagerfläche 48 an dem zweiten Kettenglied 40 der ersten Kette 23 korrespondierende Lagerfläche an der ersten Welle 80 oder an dem Stift 81
- 87: zu der Lagerfläche 78 an dem Abstandsbauteil 70 korrespondierende Lagerfläche an der ersten Welle 80 oder an dem Stift 81
- 88: Symmetrieachse der ersten Welle 80 oder des Stifts 81 und Schwenkachse
- 89: kreisförmige Schweißnaht zwischen zweitem Kettenglied 40 der ersten Kette 23 und Stift 81
- **90**: **zweite Welle** oder Stift zur gelenkigen Verbindung zweier Kettenglieder 50, 60 der zweiten Kette 25 und des zweiten Endes 72 eines Abstandsbauteils 70
- 95: zu der Lagerfläche 59 an dem ersten Kettenglied 50 der zweiten Kette 25 korrespondierende Lagerfläche an der zweiten Welle 90
- 96: zu der Lagerfläche 69 an dem zweiten Kettenglied 60 der zweiten Kette 25 korrespondierende Lagerfläche an der zweiten Welle 90
- 97: zu der Lagerfläche 79 an dem Abstandsbauteil 70 korrespondierende Lagerfläche an der zweiten Welle 90
- 98: Achse der zweiten Welle 90 und Schwenkachse
- 99: kreisförmige Schweißnaht zwischen zweitem Kettenglied 60 der zweiten Kette 25 und zweiter Welle 90

## Patentansprüche

1. **Adaptiver Spatel (20)** für ein Laryngoskop (10), mit:
einem **proximalen Ende (21);**
einem **distalen Ende (29);**
einer mit dem proximalen Ende (21) und dem distalen Ende (29) des adaptiven Spatels (20) verbundenen **ersten Kette (23)** aus einer Mehrzahl von **Kettengliedern (30, 40),** die jeweils paarweise gelenkig verbunden sind;
einer mit dem proximalen Ende (21) und dem distalen Ende (29) des adaptiven Spatels (20) verbundenen **zweiten Kette (25)** aus einer Mehrzahl von **Kettengliedern (50, 60),** die jeweils paarweise gelenkig verbunden sind;
einem **Abstandsbauteil (70),** wobei ein erstes Ende (71) des Abstandsbauteils (70) gelenkig mit einem der Kettenglieder (30, 40) der ersten Kette 23 verbunden ist, und wobei ein zweites Ende (72) des Abstandsbauteils (70) gelenkig mit einem der Kettenglieder (50, 60) der zweiten Kette (25) verbunden ist,
wobei eine oder mehrere oder alle der gelenkigen Verbindungen zwischen den Kettengliedern (30, 40) der ersten Kette (23), den Kettengliedern (50, 60) der zweiten Kette (25) und dem Abstandsbauteil (70) ein **formschlüssiges Gelenk (38, 48, 78, 80, 81, 83, 84, 87; 59, 69, 79, 90, 95, 96, 97**) umfassen wobei der adaptive Spatel ein distales Endstück (28) umfasst, das das distale Ende (29) des adaptiven Spatels (20) bildet.

2. Adaptiver Spatel nach Anspruch 1, wobei der adaptive Spatel eine Mehrzahl von Abstandsbauteilen (70) aufweist, ein erstes Ende von jedem Abstandsbauteil der Mehrzahl von Abstandsbauteilen gelenkig mit einem der Mehrzahl von Kettengliedern (30, 40) der ersten Kette verbunden ist, ein zweites Ende von jedem Abstandsbauteil der Mehrzahl von Abstandsbauteilen gelenkig mit einem der Mehrzahl von Kettengliedern (50, 60) der zweiten Kette verbunden ist.

3. Adaptiver Spatel nach Anspruch 1, wobei der adaptive Spatel eine Mehrzahl von Abstandsbauteilen (70) aufweist, das erstes Ende von jedem Abstandsbauteil der Mehrzahl von Abstandsbauteilen gelenkig mit zwei benachbarten Kettenglieder der Mehrzahl von Kettengliedern (30, 40) der ersten Kette verbunden ist, das zweite Ende von jedem Abstandsbauteil der Mehrzahl von Abstandsbauteilen gelenkig mit zwei benachbarten Kettengliedern (50, 60) der Mehrzahl von Kettengliedern der zweiten Kette verbunden ist.

4. Adaptiver Spatel nach Anspruch 3, bei dem die jeweils zwischen dem ersten Ende und dem zweiten Ende gemessene Länge der Abstandsbauteile (70) vom proximalen Ende (20) des Spatels zum distalen Ende (29) des Spatels abnimmt.

5. Adaptiver Spatel (20) nach einem der vorangehenden Ansprüche, bei dem
eine gelenkige Verbindung zwischen zwei benachbarten Kettengliedern (30, 40) der ersten Kette (23) ein **formschlüssiges Gelenk (38, 48, 80, 81, 83, 84)** umfasst, eine gelenkige Verbindung zwischen zwei benachbarten Kettengliedern (50) der zweiten Kette (25) ein **formschlüssiges Gelenk (59, 69, 90, 95, 96)** umfasst.

6. Adaptiver Spatel (20) nach dem vorangehenden Anspruch, bei dem
mehrere gelenkige Verbindungen zwischen jeweils zwei benachbarten Kettengliedern (30, 40) der ersten Kette (23) jeweils ein **formschlüssiges Gelenk (38, 48, 80, 81, 83, 84)** umfassen,
mehrere gelenkige Verbindungen zwischen jeweils zwei benachbarten Kettengliedern (50) der zweiten Kette (25) jeweils ein **formschlüssiges Gelenk (59, 69, 90, 95, 96)** umfassen.

7. Adaptiver Spatel (20) nach dem vorangehenden Anspruch, bei dem
mehrere gelenkige Verbindungen zwischen Abstandsbauteilen (70) und Kettengliedern (30, 40) der ersten Kette (23) jeweils ein **formschlüssiges Gelenk (38, 48, 78, 80, 81, 83, 84, 87)** umfassen.

8. Adaptiver Spatel (20) nach einem der vorangehenden Ansprüche, bei dem
ein erstes Kettenglied (30; 50) in eine Ausnehmung (44; 63) in einem zweiten Kettenglied (40; 60) greift,
das formschlüssige Gelenk (38, 48, 78, 80, 81, 83, 84, 87; 59, 69, 79, 90, 95, 96, 97) eine in eine Bohrung (49) in dem zweiten Kettenglied (40; 60) eingesetzte Welle (81; 90) umfasst,
die Welle (81; 90) durch die Ausnehmung (44; 63) in dem zweiten Kettenglied (40; 60) und innerhalb der Ausnehmung (44; 63) durch das erste Kettenglied (30; 50) greift,
die Welle (81; 90) zumindest entweder durch eine Übermaßpassung kraftschlüssig in der Bohrung (49) in dem zweiten Kettenglied (40; 60) gehalten ist oder durch eine Schweißnaht (89; 99) mit dem zweiten Kettenglied (40; 60) verbunden ist.

9. Adaptiver Spatel (20) nach einem der vorangehenden Ansprüche, bei dem Kettenglieder (30, 40) der ersten Kette (23) im Wesentlichen plattenförmig mit **rechteckigem** oder **trapezförmigem** Grundriss sind.

10. Adaptiver Spatel (20) nach einem der vorangehenden Ansprüche, bei dem zumindest entweder ein Kettenglied (30, 40) der ersten Kette (23) oder ein Kettenglied (50, 60) der zweiten Kette (25) oder das Abstandsbauteil (70) **Metall** aufweisen.

11. Adaptiver Spatel (20) nach einem der vorangehenden Ansprüche, ferner mit:
einer **Mehrfachgelenkeinrichtung (38, 48, 78, 80, 81, 83, 84, 87),** in die eine gelenkige Verbindung zwischen zwei Kettengliedern (30, 40) der ersten Kette (23) und eine gelenkige Verbindung zwischen einem Kettenglied (30, 40) der ersten Kette (23) und dem Abstandsbauteil (70) **integriert** ist.

12. Adaptiver Spatel (20) nach dem vorangehenden Anspruch, bei dem
die Mehrfachgelenkeinrichtung eine **Welle (80)** oder einen Achszapfen oder einen Stift (81) mit einer ersten Lagerfläche (83) und einer zweiten Lagerfläche (84; 87) umfasst,
wobei die erste Lagerfläche (83) und die zweite Lagerfläche (84) Bestandteile gelenkiger Verbindungen des Abstandsbauteils (70) mit zwei Kettengliedern (30, 40) der ersten Kette (23) sind, oder
wobei die erste Lagerfläche (83) Bestandteil der gelenkigen Verbindung zwischen zwei Kettengliedern (30, 40) der ersten Kette (23) und die zweite Lagerfläche (87) Bestandteil der gelenkigen Verbindung zwischen einem Kettenglied (30, 40) der ersten Kette (23) und dem Abstandsbauteil (70) ist.

13. Adaptiver Spatel (20) nach Anspruch 11, bei dem Lagerflächen an zwei verschiedenen Kettengliedern und eine Lagerfläche an dem Abstandsbauteil **koaxial** angeordnet sind.

14. Adaptiver Spatel (20) nach einem der vorangehenden Ansprüche, ferner mit:
korrespondierenden **Anschlagflächen (34, 43)** an zwei miteinander gelenkig verbundenen Kettengliedern (30, 40, 50, 60);
wobei die korrespondierenden Anschlagflächen (34, 43) so angeordnet sind, dass ein mechanischer Kontakt der korrespondierenden Anschlagflächen (34, 43) eine Schwenkbewegung der zwei benachbarten Kettenglieder (30, 40, 50, 60) relativ zu einander begrenzt.

15. **Adaptives Laryngoskop (10)** oder adaptive Intubationsvorrichtung, mit:
einem adaptiver **Spatel (20)** nach einem der vorangehenden Ansprüche;
einem **Griffbauteil (11),** das mit dem proximalen Ende (21) des adaptiver Spatels (20) mechanisch verbindbar oder verbunden ist.

## Claims

1. Adaptive blade (20) for a laryngoscope (10), comprising:
a proximal end (21);
a distal end (29);
a first chain (23) connected to the proximal end (21) and the distal end (29) of the adaptive blade (29) and composed of a plurality of chain links (30,40) which are each connected in pairs in an articulated manner;
a second chain (25) connected to the proximal end (21) and the distal end (29) of the adaptive blade (20) and composed of a plurality of chain links (50, 60) which are each connected in pairs in an articulated manner;
a spacer component (70), wherein a first end (71) of the spacer component (70) is connected in an articulated manner to one of the chain links (30,40) of the first chain (23), and wherein a second end (72) of the spacer component (70) is connected in an articulated manner to one of the chain links (50,60) of the second chain (25),
wherein one or more or all of the articulated connections between the chain links (30,40) of the first chain (23), the chain links (50,60) of the second chain (25) and the spacer component (70) comprise a form-fit hinge (38,48,78,80,81, 83,84,87;59,69,79,90,95,96,97), wherein the adaptive blade comprises a distal end piece (28) which forms the distal end (29) of the adaptive blade (20)

2. Adaptive blade according to Claim 1, wherein
the adaptive blade has a plurality of spacer components (70),
a first end of each spacer component of the plurality of spacer components is connected in an articulated manner to one of the plurality of chain links (30,40) of the first chain,
a second end of each spacer component of the plurality of spacer components is connected in an articulated manner to one of the plurality of chain links (50,60) of the second chain.

3. Adaptive blade according to Claim 1, wherein
the adaptive blade has a plurality of spacer components (70),
the first end of each spacer component of the plurality of spacer components is connected in an articulated manner to two adjacent chain links of the plurality of chain links (30,40) of the first chain,
the second end of each spacer component of the plurality of spacer components is connected in an articulated manner to two adjacent chain links (50,60) of the plurality of chain links of the second chain.

4. Adaptive blade according to Claim 3, in which the length of the spacer components (70) measured in each case between the first end and the second end decreases from the proximal end (20) of the blade to the distal end (29) of the blade.

5. Adaptive blade (20) according to one of the preceding claims , in which
an articulated connection between two adjacent chain links (30,40) of the first chain (23) comprises a form-fit hinge (38,48,80,81,83,84),
an articulated connection between two adjacent chain links (50) of the second chain (25) comprises a form-fit hinge (59,69,90,95,96).

6. Adaptive blade (20) according to the preceding claim, in which
several articulated connections between in each case two adjacent chain links (30,40) of the first chain (23) each comprise a form-fit hinge (38,48,80,81,83,84),
several articulated connections between in each case two adjacent chain links (50) of the second chain (25) each comprise a form-fit hinge (59,69,90,95,96).

7. Adaptive blade (20) according to the preceding claim , in which
several articulated connections between spacer components (70) and chain links (30,40) of the first chain (23) each comprise a form-fit hinge (38,48,78,80,81,83,84,87).

8. Adaptive blade (20) according to one of the preceding claims, in which a first chain link (30;50) engages in a recess (44;63) in a second chain link (40;60),
the form-fit hinge (38,48,78,80,81,83,84,87;59, 69,79,90,95,96,97) comprises a shaft (81;90) inserted into a bore (49) in the second chain link (40;60),
the shaft (81;90) engages through the recess (44;63) in the second chain link (40;60) and inside the recess (44;63) through the first chain link (30;50),
the shaft (81;90) is at least either held with force-fit engagement in the bore (49) in the second chain link (40;60) by an interference fit or is connected to the second chain link (40;60) by a weld seam (89;99).

9. Adaptive blade (20) according to one of the preceding claims, in which chain links (30,40) of the first chain (23) are substantially plate-shaped with a rectangular or trapezoidal contour.

10. Adaptive blade (20) according to one of the preceding claims, in which at least either a chain link (30, 40) of the first chain (23) or a chain link (50, 60) of the second chain (25) or the spacer component (70) has metal.

11. Adaptive blade (20) according to one of the preceding claims, further comprising:
a multi-hinge mechanism (38,48,78,80,81,83,84,87) which integrates an articulated connection between two chain links (30,40) of the first chain (23) and an articulated connection between a chain link (30,40) of the first chain (23) and the spacer component (70).

12. Adaptive blade (20) according to the preceding claim, in which
the multi-hinge mechanism comprises a shaft (20) or an axle journal or a pin (81) with a first bearing surface (83) and a second bearing surface (84;87), wherein the first bearing surface (83) and the second bearing surface (84) are constituent parts of articulated connections of the spacer component (70) to two chain links (30,40) of the first chain (23), or
wherein the first bearing surface (83) is a constituent part of the articulated connection between two chain links (30,40) of the first chain (23) and the second bearing surface (87) is a constituent part of the articulated connection between a chain link (30,40) of the first chain (23) and the spacer component (70).

13. Adaptive blade (20) according to Claim 11, in which bearing surfaces at two different chain links and a bearing surface at the spacer component are arranged coaxially.

14. Adaptive blade (20) according to one of the preceding claims, further comprising:
corresponding abutment surfaces (34,43) at two chain links (30,40,50,60) connected to each other in an articulated manner;
wherein the corresponding abutment surfaces (34,43) are arranged such that a mechanical contact of the corresponding abutment surfaces (34,43) limits a pivoting movement of the two adjacent chain links (30,40,50,60) relative to each other.

15. Adaptive laryngoscope (10) or adaptive intubation device, comprising:
an adaptive blade (20) according to one of the preceding claims;
a handle (11), which is mechanically connectable or connected to the proximal end (21) of the adaptive blade (20).

## Revendications

1. Spatule adaptative (20) pour un laryngoscope (10), comprenant :
une extrémité proximale (21) ;
une extrémité distale (29) ;
une première chaîne (23) connectée à l'extrémité proximale (21) et à l'extrémité distale (29) de la spatule adaptative (20), constituée d'une pluralité de maillons (30, 40) qui sont connectés à chaque fois par paires de manière articulée ;
une deuxième chaîne (25) connectée à l'extrémité proximale (21) et à l'extrémité distale (29) de la spatule adaptative (20), constituée d'une pluralité de maillons (50, 60) qui sont connectés à chaque fois par paires de manière articulée ;
un composant d'espacement (70), une première extrémité (71) du composant d'espacement (70) étant connectée de manière articulée à l'un des maillons (30, 40) de la première chaîne (23), et une deuxième extrémité (72) du composant d'espacement (70) étant connectée de manière articulée à l'un des maillons (50, 60) de la deuxième chaîne (25),
une ou plusieurs, ou la totalité des liaisons articulées entre les maillons (30, 40) de la première chaîne (23), les maillons (50, 60) de la deuxième chaîne (25) et le composant d'espacement (70) comprenant une articulation par engagement positif (38, 48, 78, 80, 81, 83, 84, 87 ; 59, 69, 79, 90, 95, 96, 97), la spatule adaptative comprenant un embout distal (28) qui forme l'extrémité distale (29) de la spatule adaptative (20) .

2. Spatule adaptative selon la revendication 1, la spatule adaptative présentant une pluralité de composants d'espacement (70), une première extrémité de chaque composant d'espacement de la pluralité de composants d'espacement étant connectée de manière articulée à l'un parmi la pluralité de maillons (30, 40) de la première chaîne,
une deuxième extrémité de chaque composant d'espacement de la pluralité de composants d'espacement étant connectée de manière articulée à l'un parmi la pluralité de maillons (50, 60) de la deuxième chaîne.

3. Spatule adaptative selon la revendication 1, la spatule adaptative présentant une pluralité de composants d'espacement (70), la première extrémité de chaque composant d'espacement de la pluralité de composants d'espacement étant connectée de manière articulée à deux maillons adjacents de la pluralité de maillons (30, 40) de la première chaîne,
la deuxième extrémité de chaque composant d'espacement de la pluralité de composants d'espacement étant connectée de manière articulée à deux maillons adjacents (50, 60) parmi la pluralité de maillons de la deuxième chaîne.

4. Spatule adaptative selon la revendication 3, dans laquelle la longueur des composants d'espacement (70), mesurée à chaque fois entre la première extrémité et la deuxième extrémité, diminue depuis l'extrémité proximale (20) de la spatule jusqu'à l'extrémité distale (29) de la spatule.

5. Spatule adaptative (20) selon l'une quelconque des revendications précédentes, dans laquelle une liaison articulée entre deux maillons adjacents (30, 40) de la première chaîne (23) comprend une articulation à engagement positif (38, 48, 80, 81, 83, 84), une liaison articulée entre deux maillons adjacents (50) de la deuxième chaîne (25) comprend une articulation à engagement positif (59, 69, 90, 95, 96) .

6. Spatule adaptative (20) selon la revendication précédente, dans laquelle plusieurs liaisons articulées à chaque fois entre deux maillons adjacents (30, 40) de la première chaîne (23) comprennent à chaque fois une articulation à engagement positif (38, 48, 80, 81, 83, 84), plusieurs liaisons articulées à chaque fois entre deux maillons adjacents (50) de la deuxième chaîne (25) comprennent à chaque fois une articulation à engagement positif (59, 69, 90, 95, 96).

7. Spatule adaptative (20) selon la revendication précédente, dans laquelle plusieurs liaisons articulées entre des composants d'espacement (70) et des maillons (30, 40) de la première chaîne (23) comprennent à chaque fois une articulation à engagement positif (38, 48, 78, 80, 81, 83, 84, 87) .

8. Spatule adaptative (20) selon l'une quelconque des revendications précédentes, dans laquelle un premier maillon (30 ; 50) s'engage dans un évidement (44 ; 63) dans un deuxième maillon (40 ; 60),
l'articulation à engagement positif (38, 48, 78, 80, 81, 83, 84, 87 ; 59, 69, 79, 90, 95, 96, 97) comprend un arbre (81 ; 90) inséré dans un alésage (49) dans le deuxième maillon (40 ; 60),
l'arbre (81 ; 90) s'engage à travers l'évidement (44 ; 63) dans le deuxième maillon (40 ; 60) et à l'intérieur de l'évidement (44 ; 63) à travers le premier maillon (30 ; 50),
l'arbre (81 ; 90) est au moins soit retenu par un surdimensionnement par engagement par force dans l'alésage (49) dans le deuxième maillon (40 ; 60), soit connecté par un joint de soudure (89 ; 99) au deuxième maillon (40 ; 60).

9. Spatule adaptative (20) selon l'une quelconque des revendications précédentes, dans laquelle des maillons (30, 40) de la première chaîne (23) sont essentiellement en forme de plaque avec un pourtour rectangulaire ou trapézoïdal.

10. Spatule adaptative (20) selon l'une quelconque des revendications précédentes, dans laquelle au moins soit un maillon (30, 40) de la première chaîne (23) soit un maillon (50, 60) de la deuxième chaîne (25), soit le composant d'espacement (70) présente du métal.

11. Spatule adaptative (20) selon l'une quelconque des revendications précédentes, comprenant en outre : un dispositif d'articulation multiple (38, 48, 78, 80, 81, 83, 84, 87) dans lequel sont intégrées une liaison articulée entre deux maillons (30, 40) de la première chaîne (23) et une liaison articulée entre un maillon (30, 40) de la première chaîne (23) et le composant d'espacement (70).

12. Spatule adaptative (20) selon la revendication précédente, dans laquelle le dispositif d'articulation multiple comprend un arbre (80) ou un tourillon d'axe ou une goupille (81) avec une première surface de palier (83) et une deuxième surface de palier (84 ; 87),
la première surface de palier (83) et la deuxième surface de palier (84) font partie de liaisons articulées du composant d'espacement (70) à deux maillons (30, 40) de la première chaîne (23), ou
la première surface de palier (83) fait partie de la liaison articulée entre deux maillons (30, 40) de la première chaîne (23), et la deuxième surface de palier (87) fait partie de la liaison articulée entre un maillon (30, 40) de la première chaîne (23) et le composant d'espacement (70).

13. Spatule adaptative (20) selon la revendication 11, dans laquelle des surfaces de palier au niveau de deux maillons différents et une surface de palier au niveau du composant d'espacement sont disposées coaxialement.

14. Spatule adaptative (20) selon l'une quelconque des revendications précédentes, comprenant en outre :
des surfaces de butée correspondantes (34, 43) au niveau de deux maillons (30, 40, 50, 60) connectés l'un à l'autre de manière articulée ;
les surfaces de butée correspondantes (34, 43) étant disposées de telle sorte qu'un contact mécanique des surfaces de butée correspondantes (34, 43) limite un mouvement de pivotement l'un par rapport à l'autre des deux maillons adjacents (30, 40, 50, 60).

15. Laryngoscope adaptatif (10) ou dispositif d'intubation adaptatif, comprenant :
une spatule adaptative (20) selon l'une quelconque des revendications précédentes ;
un composant de préhension (11) qui est ou peut être connecté mécaniquement à l'extrémité proximale (21) de la spatule adaptative (20).
